Europäisches Patentamt

European Patent Office

Office européen des brevets

(19)

(11) **EP 1 438 974 A2**

(12) **EUROPEAN PATENT APPLICATION**

(43) Date of publication:
21.07.2004 Bulletin 2004/30

(51) Int Cl.7: **A61K 49/18**, A61K 49/00,
A61K 49/22, A61K 51/12,
A61K 9/127

(21) Application number: 04000310.5

(22) Date of filing: 09.01.2004

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: 10.01.2003 NO 20030117
10.07.2003 NO 20033173

(71) Applicant: **Amersham Health AS
0401 Oslo (NO)**

(72) Inventors:
• **Lokling, Knut-Egil
0683 Oslo (NO)**
• **Skurtveit, Roald
Nydalen 0401 Oslo (NO)**
• **Fossheim, Sirgid Lise
Nydalen 0401 Oslo (NO)**

(74) Representative: **Flechsler, Insa et al
Amersham plc,
Amersham Place
Little Chalfont, Bucks HP7 9NA (GB)**

(54) **pH-sensitive liposomes based on diacyl-glycero-phosphoethanolamine and diacyl-succinyl-glycerol**

(57)    The invention relates to pH-sensitive liposome systems, compositions comprising said pH sensitive liposome systems and the use of such compositions as contrast agents and medicaments.

Surprisingly, we have found a pH sensitive liposome system comprising

(a)  1-acyl-2-acyl-glycero-3-phosphoethanolamine and
(b)  1-acyl-2-acylsuccinylglycerol or 1-acyl-3-acylsuccinylglycerol

wherein in (a), both acyl-residues are the same or different and are saturated acyl residues that contain at least 14 C atoms or,
one acyl residue is a saturated acyl residue that contains at least 14 C atoms and the other acyl residue is a monounsaturated acyl residue that contain at least 14 C atoms and,

wherein in (b), both acyl-residues are the same or different and are saturated acyl residues that contain at least 14 C atoms or,
one acyl residue is a saturated acyl residue that contains at least 14 C atoms and the other acyl residue is a monounsaturated acyl residue that contain at least 14 C atoms, that could be used for the preparation of pH sensitive MR contrast agents that are suitable for *in vivo* applications.

Printed by Jouve, 75001 PARIS (FR)

**Description**

The invention relates to pH-sensitive liposome systems, compositions comprising said pH sensitive liposome systems and the use of such compositions as contrast agents and medicaments.

It is well known that in the presence of pathology, physiological parameters such as pH may be altered. The pH is often reduced during cancer diseases in solid tumours, during cardiovascular diseases like stroke, during osteoporosis, inflammation and during certain autoimmune diseases. The lower pH values in tumour tissue have been attributed to the extensive production of lactic acid within tumour cells and its extrusion to the interstitial fluid where the acid is accumulated due to an impaired clearance. The reduced interstitial pH in tumour tissue has formed the basis for the development of *in vivo* pH measurement and methods for the detection of tumours.

Diagnostic agents, e.g. contrast agents encapsulated in pH sensitive liposomes were suggested for *in vivo* pH measurement and detection of tumour tissue. Such contrast agents are for example paramagnetic contrast agents used in MR imaging.

WO-A1-99/52505 describes the encapsulation of GdDTPA-BMA, a low molecular weight paramagnetic contrast agent used in magnetic resonance imaging (MRI), in pH sensitive liposomes composed of dipalmitoylphosphatidylethanolamine (DPPE) and palmitic acid (PA). *In vitro* investigations of said system showed that at physiological pH, the $T_1$-relaxivity ($r_1$) of said system was significantly lowered compared to that of non-liposomal GdDTPA-BMA, which was explained by an exchange limited relaxation process. Lowering the pH gave a sharp and 6-7 fold increase in $r_1$ due to liposome aggregation and subsequent leakage of encapsulated GdDTPA-BMA. However, said liposome system is rapidly destabilised in human blood and leaky at both - high and low pH. Hence, said liposome system is not suitable for *in vivo* diagnostic application.

D. Collins et al., Biochem. Biophys. Acta 1025 (1990), 234 - 242 describe a pH sensitive liposome system that is stable in human plasma. The liposome system is composed of unsaturated dioleoylphosphatidylethanolamine (DOPE) and dipalmitoylsuccinylglycerol (DPSG) or dioleoylsuccinylglycerol (DOSG). The pH sensitivity of the system was tested by encapsulation of calcein and monitoring of acid-induced calcein release. The potential of said system for *in vivo* drug delivery is discussed.

In contrary to pH sensitive liposomes used for drug delivery, pH sensitive liposomes that are used for the encapsulation of paramagnetic contrast agents like GdDTPA-BMA must fulfil certain additional criteria. As a change in $r_1$ is caused by an increased water access to said contrast agents the liposome membrane must be rigid enough to reduce bulk water access to the contrast agent at physiological pH. At lower pH, the increased access of bulk water may be due to, for instance, an increase of water permeability of the liposome membrane or by liposome destabilisation and subsequent leakage of the contrast agent.

pH sensitive liposomes showing a high water permeability of their membrane already at physiological pH are not suitable for the preparation of pH sensitive paramagnetic MR contrast agents for *in vivo* applications. K.-E. Løkling et al., Magn. Reson. Imaging 19 (2001), 731-738 describe that GdDTPA-BMA encapsulated in unsaturated DOPE/OA liposomes is not a suitable pH sensitive contrast agent for MR imaging due to the high water permeability of the liposome membrane at normal physiological pH. Thus, upon lowering the pH, only a poor pH/$r_1$- dependence was observed.

Accordingly, there was a need for pH sensitive MR contrast agents that are suitable for *in vivo* applications.

Surprisingly, we have found a pH sensitive liposome system comprising

  (a) 1-acyl-2-acyl-glycero-3-phosphoethanolamine and
  (b) 1-acyl-2-acylsuccinylglycerol or 1-acyl-3-acylsuccinylglycerol

wherein in (a), both acyl-residues are the same or different and are saturated acyl residues that contain at least 14 C atoms or,
one acyl residue is a saturated acyl residue that contains at least 14 C atoms and the other acyl residue is a monounsaturated acyl residue that contain at least 14 C atoms and,
wherein in (b), both acyl-residues are the same or different and are saturated acyl residues that contain at least 14 C atoms or,
one acyl residue is a saturated acyl residue that contains at least 14 C atoms and the other acyl residue is a monounsaturated acyl residue that contain at least 14 C atoms, that could be used for the preparation of pH sensitive MR contrast agents that are suitable for *in vivo* applications.

Said pH sensitive liposome system is for instance suitable for the encapsulation of paramagnetic MR contrast generating species. As such, pH sensitive liposomes are obtained which are stable and remain pH sensitive in human blood. Said liposomes are suitable for *in vivo* detection of low pH areas (i.e. areas with pH lower than normal physiological pH) and for *the in vivo* detection of tumours or other diseases like cardiovascular diseases, e.g. stroke, osteoporosis or inflammation in human or non-human animal subjects.

The liposome system according to the invention comprises compounds (a) and (b) each compound comprising two acyl residues that contain at least 14 C atoms, preferably 14 to 22 C atoms and more preferably 14 to 18 C atoms.

In one embodiment, the acyl residues in compound (a) are both saturated acyl residues that contain at least 14 C atoms and said acyl residues may be the same like for instance in dipalmitoyl-glycero-3-phosphatidyethanolamine or different like for instance in myristoyl- palmitoyl-glycero-3-phosphoethanolamine, preferably they are the same. In a preferred embodiment, said residues contain 14 to 22 C atoms, more preferably 14 to 20 C atoms. Preferred compounds (a) are dimyristoyl-glycero-3-phosphoethanolamine, dipentadecanoyl-glycero-3-phosphoethanolamine, dipalmitoyl-glycero-3-phospho-ethanolamine, diheptadecanoyl-glycero-3-phosphoethanolamine, distearoyl-glycero-3-phospho-ethanolamine, dinonadecanoyl-glycero-3-phosphoethanolamine and diarachidoyl-glycero-3-phosphoethanolamine. More preferred compounds (a) are dimyristoyl-glycero-3-phosphoethanolamine, dipalmitoyl-glycero-3-phospho-ethanolamine and distearoyl-glycero-3-phosphoethanolamine.

In another embodiment, the acyl residues in compound (a) are one saturated acyl residue that contains at least 14 C atoms, like for instance myristoyl, palmitoyl or stearoyl and one monounsaturated acyl residue that contains at least 14 C atoms, like for instance myristoleoyl, palmitoleoyl, oleoyl, elaidoyl or eicosenoyl. In a preferred embodiment, said residues contain 14 to 22 C atoms, more preferably 14 to 18 C atoms. Preferred compounds (a) are myristoyl-myristoleoyl-glycero-3-phosphoethanolamine, myristoyl-myristelaidoyl-glycero-3-phosphoethanolamine, myristoyl-palmitoleoyl-glycero-3-phosphoethanolamine, myristoyl-palmitelaidoyl-glycero-3-phosphoethanolamine, myristoyl-oleoyl-glycero-3-phosphoethanolamine, myristoylelaidoyl-glycero-3-phosphoethanolamine, palmitoyl-myristoleoyl-glycero-3-phosphoethanolamine, palmitoyl-myristelaidoyl-glycero-3-phosphoethanolamine, palinitoyl-palmitoleoyl-glycero-3-phosphoethanolamine, palmitoyl-palmitelaidoylglycero-3-phosphoethanolamine, palmitoyl-oleoyl-glycero-3-phosphoethanolamine, palmitoyl-elaidoyl-glycero-3-phosphoethanolamine, palmitoyl-eicosenoyl-glycero-3-phosphoethanolamine, stearoyl-myristoleoyl-glycero-3-phosphoethanolamine, stearoyl-myristelaidoyl-glycero-3-phosphoethanolamine, stearoyl-palmitoleoylglycero-3-phosphoethanolamine, stearoyl-palmitelaidoyl-glycero-3-phosphoethanolamine, stearoyl-oleoyl-glycero-3-phosphoethanolamine, stearoyl-elaidoylglycero-3-phosphoethanolamine, stearoyl-eicosenoyl-glycero-3-phosphoethanolamine, arachidoyl-palmitoleoyl-glycero-3-phosphoethanolamine, arachidoyl-palmitelaidoyl-glycero-3-phosphoethanolamine, arachidoyl-oleoylglycero-3-phosphoethanolamine, arachidoyl-elaidoyl-glycero-3-phosphoethanolamine and arachidoyl-eicosenoyl -glycero-3-phosphoethanolamine. More preferred compounds (a) are palmitoyl-palmitoleoyl-glycero-3-phosphoethanolamine, palmitoyl-palmitelaidoyl-glycero-3-phosphoethanolamine, palmitoyl-oleoyl-glycero-3-phosphoethanolamine, palmitoyl-elaidoyl-glycero-3-phosphoethanolamine, stearoyl-palmitoleoyl-glycero-3-phosphoethanolamine, stearoyl-palmitelaidoyl -glycero-3-phosphoethanolamine, stearoyl-oleoyl -glycero-3-phosphoethanolamine, stearoyl-elaidoyl -glycero-3-phosphoethanolamine and stearoyl-eicosenoyl -glycero-3-phosphoethanolamine. Preferably, the acyl residues in compound (a) are both saturated acyl residues that contain at least 14 C atoms. More preferably, said acyl residues are the same.

In a particularly preferred embodiment, compound (a) in the system according to the invention is partially substituted by the corresponding polyethylene glycol (PEG)-modified compound, i.e. the system comprises 1-acyl-2-acyl-glycero-3-phosphoethanolamine (a), 1-acyl-2-acyl-glycero-3-phosphoethanolamine-PEG (a*) and 1-acyl-2-acylsuccinylglycerol or 1-acyl-3-acylsuccinylglycerol (b).

It was found that the pH sensitivity (maximum r1 - minimum r1) decreased as a function of mole% (a*) in the liposome system. However, the blood circulation time can be prolonged when compounds (a*) are present in the system. Hence a compromise is necessary between long blood residence time and suitable pH sensitivity of the system. The amount of (a*) should be optimised to ensure that both the blood residence time and the pH sensitivity are suitable. Preferably, (a*) is present in the system in an amount of from 0.5 to 5 mol%, more preferably in an amount of from 1 to 3 mol%, most preferably in an amount of 1.25 to 2 mol%.

The chain length of the PEG-moiety is not critical, conveniently, commercially available PEG with a chain length of from 350 to 10000, preferably from 1000 to 5000 and more preferably form 1500 to 3000 is used. PEG as well as PEG-derivatives such as monomethoxy-PEG (mPEG) are suitable in the context of the invention. Suitably, the PEG moiety is covalently attached to the terminal amine group of (a), e.g. via an amide linkage or a carbamate linkage. Preferred compounds (a*) are the PEG-substituted compounds mentioned on page 3, line 23 to page 4, line 2 and the PEG-substituted compounds mentioned on page 4, lines 4 to page 5, line 2.

The pH sensitive liposome system according to the invention further comprises 1-acyl-2-acylsuccinylglycerol or 1-acyl-3-acylsuccinylglycerol as the compound (b). Preferably, the system according to the invention comprises 1-acyl-2-acylsuccinylglycerol as the compound (b). If not further specified, exemplified compounds (b) mean both, 1-acyl-2-acylsuccinylglycerol and 1-acyl-3-acylsuccinylglycerol.

In one embodiment, the acyl residues in compound (b) are both saturated acyl residues that contain at least 14 C atoms and said acyl residues may be the same, like for instance in dipalmitoylsuccinylglycerol or different, like for instance in palmitoyl-stearoylsuccinylglycerol, preferably they are the same. In a preferred embodiment, said residues contain 14 to 22 C atoms, more preferably 14 to 18 C atoms. Preferred compounds (b) are dimyristoylsuccinylg-lycerol, dipentadecanoylsuccinylglycerol, dipalmitoylsuccinylglycerol, diheptadecanoylsuccinylglycerol, distearoylsuc-

cinylglycerol, dinonadecanoylsuccinylglycerol and diarachidonylsuccinylglycerol. More preferred compounds (b) are dimyristoylsuccinylglycerol, dipalmitoylsuccinylglycerol and distearylsuccinylglycerol.

In another embodiment, the acyl residues in compound (b) are one saturated acyl residue that contains at least 14 C atoms, like for instance myristoyl, palmitoyl or stearoyl and one monounsaturated acyl residue that contains at least 14 C atoms, like for instance myristoleoyl, palmitoleoyl, oleoyl, elaidoyl or eicosenoyl. In a preferred embodiment, said residues contain 14 to 22 C atoms, more preferably 14 to 18 C atoms. Preferred compounds (b) are myristoyl-myristoleoyl-succinylglycerol, myristoyl-myristelaidoyl-succinylglycerol, myristoyl-palmitoleoyl-succinylglycerol, myristoyl-palmitelaidoyl-succinylglycerol, myristoyl-oleoyl-succinylglycerol, myristoyl-elaidoyl-succinylglycerol, palmitoyl-myristoleoyl-succinylglycerol, palmitoyl-myristelaidoyl-succinylglycerol, palmitoyl-palmitoleoyl-succinylglycerol, palmitoyl-palmitelaidoyl-succinylglycerol, palmitoyl-oleoyl-succinylglycerol, palmitoyl-elaidoyl-succinylglycerol, palmitoyl-eicosenoyl-succinylglycerol, stearoyl-myristoleoyl-succinylglycerol, stearoyl-myristelaidoyl-succinylglycerol, stearoyl-palmitoleoyl-succinylglycerol, stearoyl-palmitelaidoyl-succinylglycerol, stearoyl-oleoyl-succinylglycerol, stearoyl-elaidoyl-succinylglycerol, stearoyl-eicosenoyl-succinylglycerol, arachidoyl-palmitoleoyl-succinylglycerol, arachidoyl-palmitelaidoyl-succinylglycerol, arachidoyl-oleoyl-succinylglycerol, arachidoyl-elaidoyl-succinylglycerol and arachidoyl-eicosenoyl-succinylglycerol. More preferred compounds (b) are palmitoyl-palmitoleoyl-succinylglycerol, palmitoyl-palmitelaidoyl-succinylglycerol, palmitoyl-oleoyl-succinylglycerol, palmitoyl-elaidoyl-succinylglycerol, stearoyl-palmitoleoyl-succinylglycerol, stearoyl-palmitelaidoyl-succinylglycerol, stearoyl-oleoyl-succinylglycerol, stearoyl-elaidoyl-succinylglycerol and stearoyl-eicosenoyl -succinylglycerol.

Particularly preferred pH sensitive liposome systems according to the invention comprise dimyristoyl-glycero-3-phosphoethanolamine and 1,2-dimyristoylsuccinyl-glycerol, dipalmitoyl-glycero-3-phospho-ethanolamine and 1,2-dimyristoylsuccinyl-glycerol, distearoyl-glycero-3-phosphoethanolamine and 1,2-dimyristoylsuccinyl-glycerol, dimyristoyl-glycero-3-phosphoethanolamine and 1,2-dipalmitoylsuccinyl-glycerol, dipalmitoyl-glycero-3-phospho-ethanolamine and 1,2-dipalmitoylsuccinyl-glycerol, distearoyl-glycero-3-phosphoethanolamine and 1,2-dipalmitoylsuccinyl-glycerol, dimyristoyl-glycero-3-phosphoethanolamine and 1,2-distearoylsuccinyl-glycerol, dipalmitoyl-glycero-3-phospho-ethanolamine and 1,2-distearoylsuccinyl-glycerol, distearoyl-glycero-3-phosphoethanolamine and 1,2-distearoylsuccinyl-glycerol.

Further particularly preferred pH sensitive liposome systems according to the invention comprise dimyristoyl-glycero-3-phosphoethanolamine, dimyristoyl-glycero-3-phosphoethanolamine-PEG and 1,2-dimyristoylsuccinyl-glycerol, dipalmitoyl-glycero-3-phospho-ethanolamine, dipalmitoyl-glycero-3-phosphoethanolamine-PEG and 1,2-dimyristoylsuccinyl-glycerol, distearoyl-glycero-3-phosphoethanolamine, distearoyl-glycero-3-phosphoethanolamine-PEG and 1,2-dimyristoylsuccinyl-glycerol, dimyristoyl-glycero-3-phosphoethanolamine, dimyristoyl-glycero-3-phosphoethanolamine-PEG and 1,2-dipalmitoylsuccinyl-glycerol, dipalmitoyl-glycero-3-phospho-ethanolamine, dipalmitoyl-glycero-3-phospho-ethanolamine-PEG and 1,2-dipalmitoylsuccinyl-glycerol, distearoyl-glycero-3-phosphoethanolamine, distearoyl-glycero-3-phosphoethanolamine-PEG and 1,2-dipalmitoylsuccinyl-glycerol, dimyristoyl-glycero-3-phosphoethanolamine, dimyristoyl-glycero-3-phosphoethanolamine-PEG and 1,2-distearoylsuccinyl-glycerol, dipalmitoyl-glycero-3-phospho-ethanolamine, dipalmitoyl-glycero-3-phospho-ethanolamine-PEG and 1,2-distearoylsuccinyl-glycerol, distearoyl-glycero-3-phosphoethanolamine, distearoyl-glycero-3-phosphoethanolamine-PEG and 1,2-distearoylsuccinyl-glycerol.

In the pH sensitive liposome system according to the invention, compound (a) or the mixture of compounds (a) and (a*) is preferably used in excess compared to compound (b).

The pH sensitive liposome system according to the invention is stable in blood, blood plasma and in the presence of blood proteins at normal physiological pH (pH 7.4 at 37 °C) and higher pH values. Upon exposure of the pH sensitive liposome system to a low pH environment, i.e. lower than the normal physiological pH, liposomal destabilisation and leakage of the encapsulated material occurs.

Another aspect of the invention is a method for the preparation of the inventive pH sensitive liposomes. Several methods for the preparation of liposomes are known in the art and said methods may also be used for the preparation of the pH sensitive liposomes according to the invention (see for example D. D. Lasic et al., Preparation of liposomes. In D.D. Lasic (ed), Liposomes from physics to applications, Amsterdam, Elsevier Science Publishers B.V., The Netherlands, 1993, page 63 - 107.) Methods known to the skilled artisan include for example the thin film hydration method and the reverse phase evaporation method.

In a preferred embodiment, the pH sensitive liposomes according to the invention are prepared by the thin film hydration method. Briefly, a chloroform/methanol solution of the compounds (a) and (b) is rotary evaporated to dryness and the resulting film is further dried under vacuum (see for example D.D. Lasic, Preparation of liposomes. In D.D. Lasic (ed), Liposomes from physics to applications, Amsterdam, Elsevier Science Publishers B.V., The Netherlands, 1993, p. 67-73).

The pH sensitive liposomes according to the invention may be unilamellar or multilamellar liposomes. Unilamellar liposomes are preferred for the encapsulation of contrast generating species useful in MR imaging or spectroscopy, as described later in this application.

Methods for the preparation of compounds (a), (a*) and (b) are known in the art. Compounds (a) may be synthesised as described in J.T. Mason et al., Biochemistry 29 (1990), 590-598. Briefly, mono-acid or mixed-acid diacylphosphatidylcholines (for synthesis see for example J.T. Mason et al., Anal. Biochem. 113 (1981a), 96) are converted to the corresponding phosphoethanolamines by phospholipase D mediated transphosphatidylation.

Compounds (a*) may be synthesised by covalently attaching a PEG moiety (for instance carboxy-PEG) to the terminal amine group of (a), e.g. via an amide linkage or a carbamate linkage. The linkage of carboxy-PEG to the terminal amine group of phosphatidylaethanolamines is described in US 4,426,330. The carbamate linkage is for instance described in K. Ng et al., Int. J. Pharm. 193:157-166 (200).

Compounds (b) may be synthesised as described in D. Collins et al., Biochem. Biophys. Acta 1025 (1990), 234 - 242. Briefly, 1,2- or 1,3-diacylglycerols (for the synthesis of mono-acid or mixed acid diacylglycerols see for example D.R. Kodali et al., Chem. Phys. Lipids 61(2), 1992, 169-173) are combined with succinate anhydride and triethylamine in chloroform. The mixture is incubated at 37 °C overnight and then subjected to water extraction to remove hydrophilic contaminants and triethylamine.

Yet another aspect of the invention are compositions comprising the pH sensitive liposome system according to the invention and at least one contrast generating species useful in MR imaging or spectroscopy, or optical imaging. Such compositions could be used as contrast agents in MR imaging or spectroscopy and optical imaging.

The term "contrast generating species" is used herein to refer to a chemical entity or a chemical composition which generate a contrast effect when used in imaging modalities.

Suitable contrast generating species in light imaging or optical imaging are dyes like for instance fluorescent dyes, near infrared dyes or UV dyes. Compositions comprising the inventive pH sensitive liposome systems and dyes can be used as pH sensitive contrast agents in optical imaging or light imaging. For instance, dyes are encapsulated in the inventive pH sensitive liposome system and the composition obtained is administered to a human or non-human animal subject. Upon exposure to an environment with lower pH (e.g. tumour tissue), the pH sensitive liposome system becomes leaky and the dye will be released. Said release will be detectable by *in vivo* light imaging or optical imaging, thus allowing the detection of e.g. tumour tissue.

In a preferred embodiment, the inventive compositions comprise dyes which are encapsulated in the inventive pH sensitive system and which - upon release at lower pH - change signal intensity. Preferably, water-soluble fluorescent dyes like carboxyfluorescin or calcein are encapsulated in the inventive pH sensitive liposome systems in relatively high concentrations. At such high concentrations, said dyes are self quenching, i.e. the fluorescence is very low compared to a diluted dye sample. When the dye is released from the pH sensitive liposome system at lower pH and diluted, it will fluoresce intensely and said increase in signal intensity will be detectable in optical imaging.

In another preferred embodiment, the inventive compositions comprise dyes which are encapsulated in the inventive pH sensitive system and which - upon release at lower pH - change spectral characteristics. For example, the spectral characteristics of the dye arsenazo III change markedly in the presence or absence of calcium ions. Calcium ions bind to the dye and cause a spectral shift from 560 nm to 606/660 nm. When said dyes are encapsulated in the inventive pH sensitive system and the surrounding pH is as such that the liposomes are intact, calcium ions can not penetrate the liposome membrane and thus can not interact with the dye. When the pH sensitive liposome system becomes leaky at lower pH and the dye is released, it will bind to calcium ions, e.g. naturally present in tissue fluids, and produce a spectral shift that will be detectable in optical imaging.

Suitable contrast generating species in MR imaging or spectroscopy are paramagnetic compounds, superparamagnetic compounds like superparamagnetic iron oxide particles, ferrimagnetic or ferromagnetic compounds. Further suitable contrast generating species in MR imaging or spectroscopy compounds containing other non zero spin nuclei than hydrogen, e.g. $^{19}F$, $^{13}C$, $^{29}Si$, $^{31}P$ and certain noble gases like $^{129}Xe$ or $^3He$. Said compounds may either contain said non-zero nuclei in an abundant form or they may be isotopically enriched with said nuclei. Alternatively, such compounds may be used in their hyperpolarised form, i.e. the nuclear polarisation of the non zero spin nuclei present in said compounds is enhanced.

Preferred contrast generating species in MR imaging spectroscopy are paramagnetic compounds such as stable free radicals, compounds comprising transition metals and compounds comprising lanthanide metals. Such compounds are preferably transition metal chelates, transition metal salts and lanthanide metal chelates, e.g. manganese salts, gadolinium chelates or dysprosium chelates. Preferred compounds are gadolinium chelates like GdDTPA, GdDTPA-BMA, GdDOTA, GdHPDO3A, Pr-, Dy-, Eu- and Tm-chelates, Mn-chelates and manganese salts like $MnCl_2$. Preferably, compositions comprising the inventive pH sensitive liposome systems and paramagnetic compounds are used as pH sensitive contrast agents in MR imaging. For instance, Gd-chelates are encapsulated in the inventive pH sensitive liposome system and the composition obtained is administered to a human or non-human animal subject. At normal physiological pH, the $T_1$ relaxivity of the composition is low and, as a result, the contrast enhancement is also low. Upon exposure to an environment with lower pH (e.g. tumour tissue), the pH sensitive liposome system becomes leaky and water can access the Gd-chelate, leading to a strong increase in $T_1$ relaxivity and thus leading to a strong contrast enhancement.

Paramagnetic compounds have either an effect on the relaxation times/relaxivity (e.g. Gd-chelates) or an effect on the chemical shift (e.g. Eu-chelates). Compounds having an effect on the chemical shift may be used as shift reagents in MR spectroscopy.

In a particularly preferred embodiment, compositions according to the invention comprise the inventive pH sensitive liposome system and Gd-chelates, preferably Gd-chelates that show extended blood retention like those disclosed in WO-A-96/23526, particularly gadofosveset, which binds to albumin and shows an enhanced $r_1$ due to reduced molecular tumbling.

Such compositions can be prepared by hydrating the pH sensitive liposome system according to the invention with an aqueous solution containing the Gd-chelate (see for example K.-E. Løkling et al. Magn. Reson. Imaging 19 (2001), 731-738). Extra-liposomal Gd-chelate may be removed by dialyses. In a preferred embodiment, a relatively concentrated aqueous solution of Gd-chelate is used, preferably a solution that has the same osmolality as the blood of the human or non-human animal subject the composition is administered to. The compositions may be prepared from unilamellar or multilamellar pH sensitive liposomes, preferably from unilamellar pH sensitive liposomes. The larger the size of the unilamellar liposomes, the lower the "start-relaxivity" (i.e. the relaxivity at physiological and higher pH) and hence the larger the difference in relaxivity between low and physiological/high pH. On the other hand, small liposomes show prolonged circulation time in blood *in vivo*. Thus, those two effects have to be balanced when preparing such compositions. The circulation time of large unilamellar liposomes can be prolonged by PEG-incorporation.

Yet another aspect of the invention is a method for the identification of a tumour, inflammation or cardiac infarct tissue in a human or non-human animal subject comprising

a) administering to said human or non-human animal subject a contrast agent comprising a composition comprising the pH sensitive liposome system according to the invention and at least one contrast generating species useful in MR imaging or spectroscopy or optical imaging and
b) generating at least one MR image, optical image or MR spectrum of said subject.

Contrast agents comprising a composition comprising the pH sensitive liposome system according to the invention and at least one contrast generating species useful in MR imaging or spectroscopy or optical imaging are preferably formulated in conventional pharmaceutical or veterinary parenteral administration forms, e.g. suspensions, dispersions, etc., for example in an aqueous carrier medium such as water for injections. If an aqueous carrier medium is used, the osmolality of the carrier medium is preferably the same as the osmolality of the intraliposomal solution. Said contrast agents may further contain pharmaceutically acceptable diluents and excipients and formulation aids, for example stabilisers, antioxidants, osmolality adjusting agents, buffers or pH-adjusting agents. The most preferred formulation for said contrast agents is a sterile solution or suspension for intravascular administration or for direct injection into area of interest. Where said contrast agents are formulated in a ready-to-use form for parenteral administration, the carrier medium is preferably isotonic or somewhat hypertonic.

The dosage of said contrast agents depends upon the clinical indication, the contrast generating species and the means by which contrast enhancement occurs.

The contrast agents may be administered in different ways with parenteral administration being the preferred embodiment. The contrast agents may be administered into the vasculature or directly into an organ or muscle tissue. Intravenous administration is particularly preferred.

Yet another aspect of the invention is the use of a composition comprising the pH sensitive liposome system according to the invention and at least one contrast generating species useful in MR imaging or spectroscopy or optical imaging for the manufacture of a contrast agent for the identification of a tumour, inflammation or cardiac infarct tissue in a human or non-human animal subject by MR imaging, optical imaging or MR spectroscopy. Further, it is possible to map pH in tumours and use the information obtained to characterise tumours according to their pH. This is particularly useful in choosing the adequate treatment of a tumour. It is for instance known that radiation therapy is minor efficient in tumours with a low pH than in tumours with a higher pH.

Yet another aspect of the invention are compositions comprising the pH sensitive liposome system according to the invention and at least one therapeutic agent. Preferred therapeutic agents are agents useful in tumour therapy and therapeutic agents useful in the treatment of inflammation.

The encapsulation of therapeutic agents in the pH sensitive liposome system according to the invention and the administration of the compositions obtained to a patient has several advantages: circulation lifetime of the therapeutic agents is enhanced by encapsulation into the inventive system which is stable at normal physiological pH. The therapeutic agent may be encapsulated in a high concentration and released at exactly that site in the body where therapy should take place.

Thus, a therapeutic agent used in tumour therapy could be encapsulated in the pH sensitive liposome system according to the invention. Upon administration to the patient, the liposomes will circulate in the blood system. Tumours are known to have abnormal capillary permeability, i.e. the capillary walls are "leaky". Prolonged circulation of the liposomes in the blood stream will result in an extravasation of the liposomes into the tumour interstitium where the pH is lower than the normal physiological pH. The pH sensitive liposome system according to the invention becomes leaky and the therapeutic agent encapsulated therein will be released exactly at the site in the body where it should make its impact.

Another aspect of the invention are compositions comprising the pH sensitive liposome system according to the invention and at least one therapeutic agent for use as a medicament.

Yet another aspect of the invention is the use of the pH sensitive liposome system according to the invention and at least one therapeutic agent for the manufacture of a medicament for the treatment of tumours or inflammation.

Yet another aspect of the invention are compositions comprising the pH sensitive liposome system according to the invention, at least one contrast generating species useful in MR imaging or spectroscopy, optical imaging, X-ray, PET or ultrasound imaging and at least one therapeutic agent. Such compositions can be used for monitoring the delivery of therapeutic agents *in vivo*.

Another aspect of the invention are compositions comprising the pH sensitive liposome system according to the invention, at least one contrast generating species useful in MR imaging or spectroscopy, optical imaging, X-ray, PET or ultrasound imaging and at least one therapeutic agent for use as medicaments.

### Examples:

### Example 1

### Preparation of a pH-sensitive liposome system according to the invention:

### 1.a Preparation of lipid thin film

Blends of 1-acyl-2-acyl-glycero-3-phosphoethanolamine and 1-acyl-2-acylsuccinyl-glycerol or 1-acyl-3-acylsuccinylglycerol were used and liposomes were prepared by the thin film hydration method. Briefly, a 10/1 (v/v) chloroform/methanol solution of the lipids was rotary evaporated to dryness and the resulting film was further dried under vacuum over night. Liposome systems according to the invention comprising PEG-modified lipids (a*) were synthesised in the same way.

### 1.b Preparation of pH-sensitive paramagnetic liposomes

The product of 1.a was hydrated with an aqueous solution containing 250 mM GdDTPA-BMA and 50 mM TRIS/HCl buffer (pH 8.4), giving a total lipid concentration of 25 mg/ml. The liposomes were sized down by ultrasonic processing (W-385 sonicator, Heat systems — Ultrasonics inc., Farmingdale, NY, USA) and/or sequential extrusion (Lipex Extruder, Lipex Biomembranes Inc., Vancouver, B.C., Canada) through polycarbonate filters (Millipore Co., Bedford, MA, USA) with various pore diameters. The lipid hydration, sonication and/or extrusion procedures were performed well above the gel-to-liquid crystalline phase transition temperature ($T_m$) of the lipid membrane. Untrapped metal chelate was removed by dialysis (Spectra/For membrane tubing MW cutoff 50000, Spectrum Medical Industries Inc., Houston, TX, USA) against isosmotic and isoprotic glucose solution.

### 1.c Physicochemical characterisation

The osmolality was determined by freezing point depression osmometry (Fiske one-ten osmometer, Fiske Associates, Norwood, MA, USA). For the size distribution measurements, liposomes were diluted with isosmotic and isoprotic glucose solution. The intensity-weighted hydrodynamic liposome diameter was determined by photon correlation spectroscopy at a scattering angle of 90° at 25 °C (Malvern PS/MW 4700, Malvern Instruments Ltd., Malvern, England). The width of the particle size distribution was expressed by the polydispersity index. The effective Gd concentration ($C_{eff}$) in the liposome preparations, defined as the Gd concentration in the total sample volume, was determined by inductively coupled plasma atomic emission spectrophotometry (ICP-AES, Perkin Elmer Optima 3300 DV, Perkin Elmer Inc., Norwalk, CT, USA). For the determination of $C_{eff}$, liposomes were diluted with a 0.2% aqueous solution of Triton X-100. Yttrium (III) was added to the samples and standard solutions to perform simultaneous internal standardisation. The Gd concentration in the samples was determined using a multipoint standard calibration curve. pH measurements were performed with a calomel reference pH electrode (Cole-Parmer Instrument Co., Vernon Hills, IL, USA) attached to a pH meter (Orion 250A, Orion Research Inc., Beverly, MA, USA).

**Example 2**

**pH - $r_1$ dependence of DPPE/DPSG (4:1 mole ratio) liposomal GdDTPA-BMA in isosmotic buffer solutions**

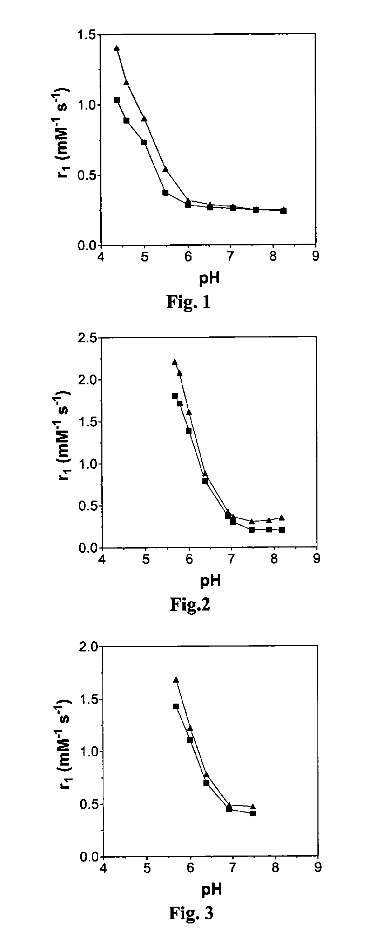

DPPE (dipalmitoyl-glycero-3-phosphatidylethanolamine) / DPSG (dipalmitoylsuccinylglycerol) liposomes (4:1 mole ratio) containing GdDTPA-BMA were prepared according to the method described in Example 1. The mean hydrodynamic diameter of the liposomes was measured to 130 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(*N*-morpholino) ethanesulfonic acid (MES) buffers). The buffered liposome dispersions were incubated at 37 °C prior to the relaxation measurements. The investigated pH-range was 4.4 - 8.3. Table 1 and Fig. 1 show the pH-$r_1$ dependence of DPPE/DPSG liposomal Gd-DTPA-BMA at 37 °C in MES buffers after 20 minutes (■) and 2.0 (▲) hours incubation.

Table 1

| pH | $r_1$ (s$^{-1}$ mM$^{-1}$) | |
| --- | --- | --- |
| | 20 minutes incubation | 2.0 hours incubation |
| 4.4 | 1.03 | 1.41 |
| 4.6 | 0.89 | 1.16 |
| 5.0 | 0.73 | 0.90 |
| 5.5 | 0.38 | 0.54 |
| 6.0 | 0.29 | 0.32 |
| 6.5 | 0.27 | 0.29 |
| 7.1 | 0.26 | 0.27 |
| 7.6 | 0.25 | 0.25 |
| 8.3 | 0.24 | 0.25 |

Table 1/ Fig. 1 shows that $r_1$ of DPPE/DPSG liposomal Gd-DTPA-BMA in isosmotic MES-buffers was relatively constant between pH 8.3 and 6.0, but increased significantly by lowering the pH from 6.0 to 4.4.

**Example 3**

**pH - $r_1$ dependence of DPPE/DPSG (4:1 mole ratio) liposomal GdDTPA-BMA in buffered human whole blood.**

DPPE/DPSG liposomes (4:1 mole ratio) containing GdDTPA-BMA were prepared according to the method described in Example 1. The mean hydrodynamic diameter of the liposomes was measured to 130 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 2 and Fig. 2 show the pH-$r_1$ dependence of DPPE/DPSG liposomal Gd-DTPA-BMA at 37 °C in MES/HEPES buffered whole blood (50%) after 20 minutes (■) and 2.0 (▲) hours incubation. The $r_1$ values are corrected for the content of solid materials present in whole blood.

Table 2

| pH | $r_1$ (s$^{-1}$ mM$^{-1}$) | |
| --- | --- | --- |
| | 20 minutes incubation | 2.0 hours incubation |
| 5.7 | 1.80 | 2.21 |
| 5.8 | 1.71 | 2.07 |
| 6.0 | 1.39 | 1.61 |
| 6.4 | 0.79 | 0.88 |

Table 2   (continued)

| pH | r$_1$ (s$^{-1}$ mM$^{-1}$) | |
|---|---|---|
| | 20 minutes incubation | 2.0 hours incubation |
| 6.9 | 0.37 | 0.42 |
| 7.0 | 0.30 | 0.36 |
| 7.5 | 0.21 | 0.31 |
| 7.9 | 0.21 | 0.32 |
| 8.2 | 0.20 | 0.35 |

Table 2/Fig. 2 shows that the r$_1$ of DPPE/DPSG liposomal Gd-DTPA-BMA in buffered human whole blood was relatively constant between pH 8.2 and 7.5, but increased significantly by lowering the pH from 7.0 to 5.5. The onset of the r$_1$ increase when lowering the pH was at a higher pH value compared to the results in Table 1/Fig. 1, and the relative r$_1$-response was higher.

**Example 4**

**pH - r$_1$ dependence of DPPE/DPSG (4:1 mole ratio) liposomal GdDTPA-BMA preincubated in human whole blood.**

DPPE/DPSG liposomes (4:1 mole ratio) containing GdDTPA-BMA were prepared according to the method described in Example 1. The mean hydrodynamic diameter of the liposomes was measured to 130 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. T$_1$-relaxation times were measured at 37 °C and 0.47 Tesla (Minispec PC-120b, Bruker GmbH, Rheinstetten, Germany). Table 3 and Fig. 3 show the pH-r$_1$ dependence of DPPE/DPSG liposomal Gd-DTPA-BMA at 37 °C after preincubation in human whole blood and subsequently further incubation with MES/HEPES buffers (and one vial with glucose) for 20 minutes (■) and 60 (▲)minutes. The r$_1$ values are corrected for the content of solid materials present in whole blood.

Table 3

| pH | r$_1$ (s$^{-1}$ mM$^{-1}$) | |
|---|---|---|
| | 20 minutes incubation | 60 minutes incubation |
| 5.7 | 1.43 | 1.68 |
| 6.0 | 1.10 | 1.22 |
| 6.4 | 0.70 | 0.78 |
| 6.9 | 0.45 | 0.49 |
| 7.5 | 0.40 | 0.47 |
| glucose* | 0.41 | 0.48 |

*Dilution with glucose instead of buffer

Table 3/Fig. 3 shows that the r$_1$ of DPPE/DPSG liposomal Gd-DTPA-BMA preincubated in human whole blood was relatively constant between pH 7.5 and 6.9, but increased significantly by lowering the pH from6.9 to 5.7. The onset of the r$_1$ increase when lowering the pH was at a slightly lower pH value compared to the results in Table 2/Fig. 2, and the relative r$_1$-response was lower.

### Example 5

### pH - r$_1$ dependence of DMPE/DPSG (9:1 mole ratio) liposomal GdDTPA-BMA in isosmotic buffer solutions

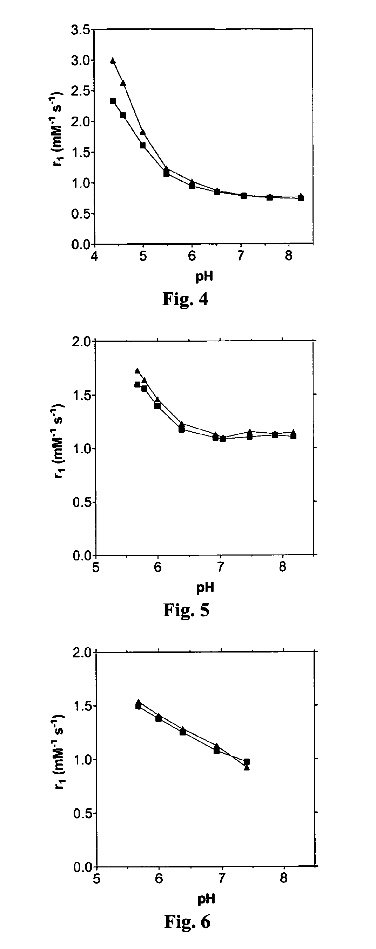

DMPE (dimyristoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 122 nm as described in Example 1. The T$_1$-relaxation times were measured at 37 °C and 0.47 Tesla (Minispec PC-120b, Bruker GmbH, Rheinstetten, Germany) in different isosmotic buffer solutions (0.05 M 2-(N-morpholino) ethanesulfonic acid (MES) buffers). The buffered liposome dispersions were incubated at 37 °C prior to the relaxation measurements. The investigated pH-range was 4.4 - 8.3. Table 4 and Fig. 4 show the pH-r$_1$ dependence of DMPE/DPSG liposomal Gd-DTPA-BMA at 37 °C in MES buffers after 20 minutes (■) and 2.0 (▲) hours incubation.

Table 4

| pH | r$_1$ (s$^{-1}$ mM$^{-1}$) | |
|---|---|---|
| | 20 minutes incubation | 2.0 hours incubation |
| 4.4 | 2,33 | 2,99 |
| 4.6 | 2,10 | 2,63 |
| 5.0 | 1,61 | 1,83 |
| 5.5 | 1,15 | 1,24 |
| 6.0 | 0,95 | 1,02 |
| 6.5 | 0,85 | 0,87 |
| 7.1 | 0,78 | 0,79 |
| 7.6 | 0,75 | 0,77 |
| 8.3 | 0,73 | 0,77 |

### Example 6

### pH - r$_1$ dependence of DMPE/DPSG (9:1 mole ratio) liposomal GdDTPA-BMA in buffered human whole blood.

DMPE (dimyristoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 122 nm as described in Example 1. The T$_1$-relaxation times were measured at 37 °C and 0.47 Tesla (Minispec PC-120b, Bruker GmbH, Rheinstetten, Germany) in different buffer solutions (0.25 M MES and 0.25 M N-2-hydroxyethylpiperazine-N'-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 5 and Fig. 5 show the pH-r$_1$ dependence of DMPE/DPSG liposomal Gd-DTPA-BMA at 37 °C in MES/HEPES buffered whole blood (50%) after 20 minutes (■) and 2.0 (▲) hours incubation. The r$_1$ values are corrected for the content of solid materials present in whole blood.

Table 5

| pH | r$_1$ (s$^{-1}$ mM$^{-1}$) | |
|---|---|---|
| | 20 minutes incubation | 2.0 hours incubation |
| 5.7 | 1,60 | 1,73 |
| 5.8 | 1,56 | 1,64 |
| 6.0 | 1,40 | 1,46 |
| 6.4 | 1,18 | 1,23 |
| 6.9 | 1,10 | 1,13 |
| 7.0 | 1,09 | 1,10 |

Table 5   (continued)

| | $r_1$ (s$^{-1}$ mM$^{-1}$) | |
|---|---|---|
| pH | 20 minutes incubation | 2.0 hours incubation |
| 7.5 | 1,11 | 1,15 |
| 7.9 | 1,12 | 1,14 |
| 8.2 | 1,11 | 1,15 |

**Example 7**

**pH - $r_1$ dependence of DMPE/DPSG (9:1 mole ratio) liposomal GdDTPA-BMA preincubated in human whole blood.**

DMPE (dimyristoyl-glycero-3-phosphatidylethanolamine) / DPSG (dipalmitoylsuccinylglycerol) liposomes (9: 1 mole ratio) containing GdDTPA-BMA were prepared according to the method described in Example 1. The mean hydrodynamic diameter of the liposomes was measured to 122 nm as described in Example 1.The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 6 and Fig. 6 show the pH-$r_1$ dependence of DMPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 6

| | $r_1$ (s$^{-1}$ mM$^{-1}$) | |
|---|---|---|
| pH | 20 minutes incubation | 60 minutes incubation |
| 5.7 | 1,57 | 1,54 |
| 6.0 | 1,45 | 1,41 |
| 6.4 | 1,32 | 1,28 |
| 6.9 | 1,14 | 1,13 |
| 7.4 glucose* | 1,03 | 0,92 |

*Dilution with glucose instead of buffer

**Example 8**

**pH - $r_1$ dependence of DMPE/DPSG (7:3 mole ratio) liposomal GdDTPA-BMA in isosmotic buffer solutions**

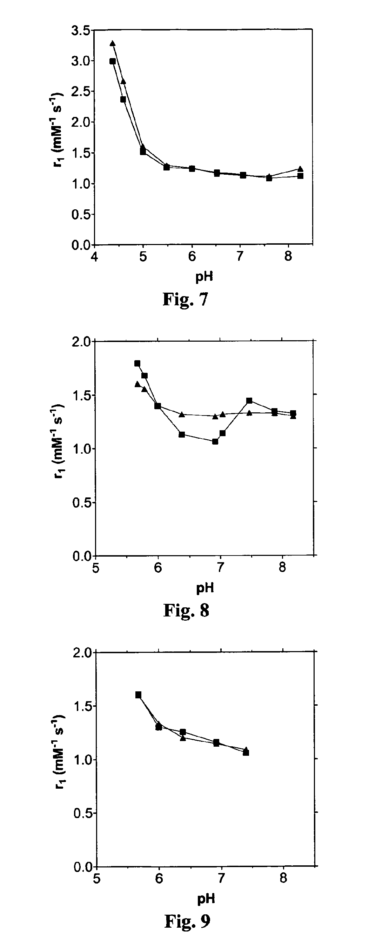

DMPE (dimyristoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 115 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(N-morpholino) ethanesulfonic acid (MES) buffers). The buffered liposome dispersions were incubated at 37 °C prior to the relaxation measurements. The investigated pH-range was 4.4 - 8.3. Table 7 and Fig. 7 show the pH-$r_1$ dependence of DMPE/DPSG liposomal Gd-DTPA-BMA at 37 °C in MES buffers after 20 minutes (■) and 2.0 (▲) hours incubation.

Table 7

| | $r_1$ (s$^{-1}$ mM$^{-1}$) | |
|---|---|---|
| pH | 20 minutes incubation | 2.0 hours incubation |
| 4.4 | 2,99 | 3,29 |

Table 7   (continued)

| | $r_1$ (s$^{-1}$ mM$^{-1}$) | |
|---|---|---|
| pH | 20 minutes incubation | 2.0 hours incubation |
| 4.6 | 2,37 | 2,66 |
| 5.0 | 1,51 | 1,61 |
| 5.5 | 1,26 | 1,29 |
| 6.0 | 1,24 | 1,25 |
| 6.5 | 1,17 | 1,15 |
| 7.1 | 1,13 | 1,12 |
| 7.6 | 1,08 | 1,10 |
| 8.3 | 1,11 | 1,22 |

**Example 9**

**pH - $r_1$ dependence of DMPE/DPSG (7:3 mole ratio) liposomal GdDTPA-BMA in buffered human whole blood.**

DMPE (dimyristoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 115 nm as described in Example 1. The $T_1$-relaxation times were measured at 37 °C and 0.47 Tesla (Minispec PC-120b, Broker GmbH, Rheinstetten, Germany) in different buffer solutions (0.25 M MES and 0.25 M $N$-2-hydroxyethylpiperazine-$N'$-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 8 and Fig. 8 show the pH-$r_1$ dependence of DMPE/ DPSG liposomal Gd-DTPA-BMA at 37 °C in MES/HEPES buffered whole blood (50%) after 20 minutes (■) and 2.0 (▲) hours incubation. The $r_1$ values are corrected for the content of solid materials present in whole blood.

Table 8

| | $r_1$ (s$^{-1}$ mM$^{-1}$) | |
|---|---|---|
| pH | 20 minutes incubation | 2.0 hours incubation |
| 5.7 | 1,60 | 1,80 |
| 5.8 | 1,56 | 1,68 |
| 6.0 | 1,40 | 1,40 |
| 6.4 | 1,32 | 1,13 |
| 6.9 | 1,30 | 1,06 |
| 7.0 | 1,32 | 1,14 |
| 7.5 | 1,33 | 1,45 |
| 7.9 | 1,33 | 1,35 |
| 8.2 | 1,30 | 1,33 |

**Example 10**

**pH - $r_1$ dependence of DMPE/DPSG (7:3 mole ratio) liposomal GdDTPA-BMA preincubated in human whole blood.**

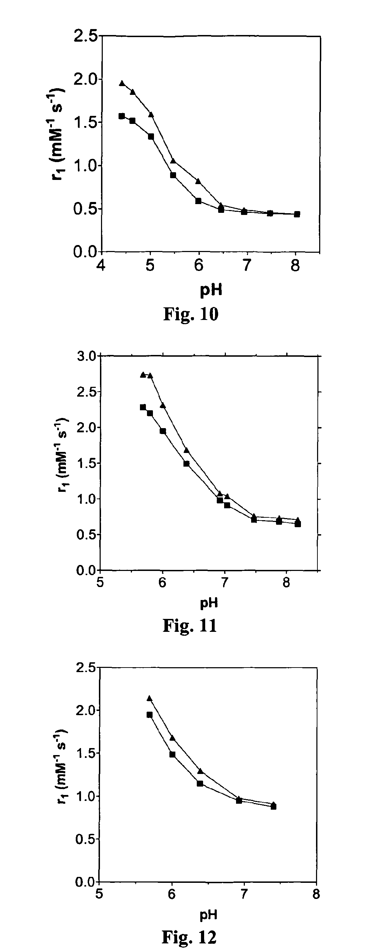

DMPE (dimyristoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 115 nm as described in Example 1.The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 9 and Fig. 9 show the pH-$r_1$ dependence of DMPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 9

| pH | $r_1$ (s$^{-1}$ mM$^{-1}$) | |
|---|---|---|
| | 20 minutes incubation | 60 minutes incubation |
| 5.7 | 1,61 | 1,60 |
| 6.0 | 1,30 | 1,33 |
| 6.4 | 1,26 | 1,20 |
| 6.9 | 1,16 | 1,14 |
| 7.4 glucose* | 1,06 | 1,09 |

*Dilution with glucose instead of buffer

## Example 11

### pH - $r_1$ dependence of DPPE/DPSG (9:1 mole ratio) liposomal GdDTPA-BMA in isosmotic buffer solutions

DPPE (dipalmitoyl-glycero-3-phosphatidylethanolamine) / DPSG (dipalmitoylsuccinylglycerol) liposomes (9: 1 mole ratio) containing GdDTPA-BMA were prepared according to the method described in Example 1. The mean hydrodynamic diameter of the liposomes was measured to 116 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(*N*-morpholino) ethanesulfonic acid (MES) buffers). The buffered liposome dispersions were incubated at 37 °C prior to the relaxation measurements. The investigated pH-range was 4.4 - 8.0. Table 10 and Fig. 10 show the pH-$r_1$ dependence of DPPE/DPSG liposomal Gd-DTPA-BMA at 37 °C in MES buffers after 20 minutes (■) and 2.0 (▲) hours incubation.

Table 10

| pH | $r_1$ (s$^{-1}$ mM$^{-1}$) | |
|---|---|---|
| | 20 minutes incubation | 2.0 hours incubation |
| 4,4 | 1,58 | 1,96 |
| 4,6 | 1,52 | 1,86 |
| 5,0 | 1,34 | 1,59 |
| 5,5 | 0,89 | 1,06 |
| 6,0 | 0,59 | 0,82 |
| 6,5 | 0,49 | 0,54 |
| 6,9 | 0,46 | 0,48 |
| 7,5 | 0,45 | 0,46 |
| 8,0 | 0,44 | 0,44 |

## Example 12

### pH - $r_1$ dependence of DPPE/DPSG (9:1 mole ratio) liposomal GdDTPA-BMA in buffered human whole blood.

DPPE (dipalmitoyl-glycero-3-phosphatidylethanolamine) / DPSG (dipalmitoylsuccinylglycerol) liposomes (9: 1 mole ratio) containing GdDTPA-BMA were prepared according to the method described in Example 1. The mean hydrodynamic diameter of the liposomes was measured to 116 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 11 and Fig. 11 show the pH-$r_1$ dependence of DPPE/DPSG liposomal Gd-DTPA-BMA at 37 °C in MES/HEPES buffered whole blood (50%) after 20 minutes (■) and 2.0 (▲) hours incubation. The $r_1$ values are corrected for the content of solid materials present in whole blood.

Table 11

| pH | r_1 (s^{-1} mM^{-1}) | |
|----|----------------------|--------------------|
|    | 20 minutes incubation | 2.0 hours incubation |
| 5.7 | 2,28 | 2,74 |
| 5.8 | 2,20 | 2,73 |
| 6.0 | 1,95 | 2,32 |
| 6.4 | 1,49 | 1,69 |
| 6.9 | 0,98 | 1,08 |
| 7.0 | 0,91 | 1,04 |
| 7.5 | 0,71 | 0,76 |
| 7.9 | 0,69 | 0,73 |
| 8.2 | 0,65 | 0,71 |

**Example 13**

**pH - $r_1$ dependence of DPPE/DPSG (9:1 mole ratio) liposomal GdDTPA-BMA preincubated in human whole blood.**

DPPE (dipalmitoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 116 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 12 and Fig. 12 show the pH-$r_1$ dependence of DPPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 12

| pH | r_1 (s^{-1} mM^{-1}) | |
|----|----------------------|-----------------------|
|    | 20 minutes incubation | 60 minutes incubation |
| 5.7 | 1,95 | 2,15 |
| 6.0 | 1,49 | 1,69 |
| 6.4 | 1,15 | 1,30 |
| 6.9 | 0,95 | 0,98 |
| 7.5 | 0,88 | 0,91 |
| 7.4 glucose* | 1,95 | 2,14 |

*Dilution with glucose instead of buffer

**Example 14**

**pH - r$_1$ dependence of DPPE/DPSG (7:3 mole ratio) liposomal GdDTPA-BMA in isosmotic buffer solutions**

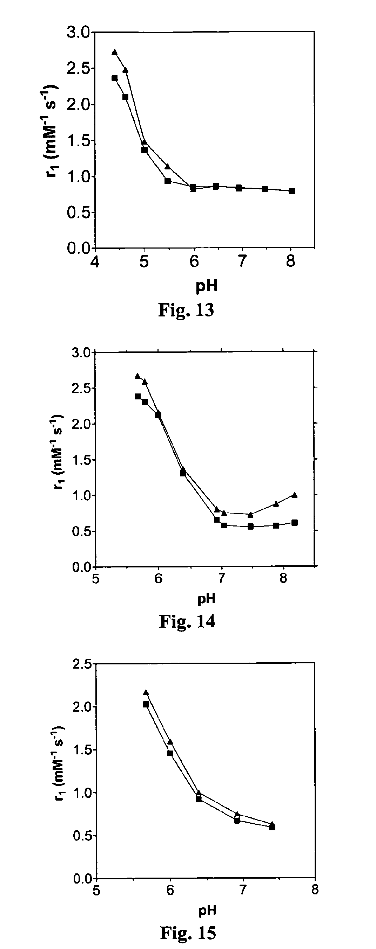

DPPE (dipalmitoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 110 nm as described in Example 1. The T$_1$-relaxation times were measured at 37 °C and 0.47 Tesla (Minispec PC-120b, Bruker GmbH, Rheinstetten, Germany) in different isosmotic buffer solutions (0.05 M 2-(*N*-morpholino) ethanesulfonic acid (MES) buffers). The buffered liposome dispersions were incubated at 37 °C prior to the relaxation measurements. The investigated pH-range was 4.4 - 8.0. Table 13 and Fig. 13 show the pH-r$_1$ dependence of DPPE/DPSG liposomal Gd-DTPA-BMA at 37 °C in MES buffers after 20 minutes (■) and 2.0 (▲) hours incubation.

Table 13

| pH | r$_1$ (s$^{-1}$ mM$^{-1}$) | |
|---|---|---|
| | 20 minutes incubation | 2.0 hours incubation |
| 4,4 | 2,37 | 2,72 |
| 4,6 | 2,10 | 2,48 |
| 5,0 | 1,36 | 1,48 |
| 5,5 | 0,93 | 1,13 |
| 6,0 | 0,85 | 0,81 |
| 6,5 | 0,86 | 0,85 |
| 6,9 | 0,83 | 0,82 |
| 7,5 | 0,81 | 0,81 |
| 8,0 | 0,78 | 0,78 |

**Example 15**

**pH - r$_1$ dependence of DPPE/DPSG (7:3 mole ratio) liposomal GdDTPA-BMA in buffered human whole blood.**

DPPE (dipalmitoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 110 nm as described in Example 1. The T$_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 14 and Fig. 14 show the pH-r$_1$ dependence of DPPE/ DPSG liposomal Gd-DTPA-BMA at 37 °C in MES/HEPES buffered whole blood (50%) after 20 minutes (■) and 2.0 (▲) hours incubation. The r$_1$ values are corrected for the content of solid materials present in whole blood.

Table 14

| pH | r$_1$ (s$^{-1}$ mM$^{-1}$) | |
|---|---|---|
| | 20 minutes incubation | 2.0 hours incubation |
| 5.7 | 2,38 | 2,66 |
| 5.8 | 2,30 | 2,59 |
| 6.0 | 2,11 | 2,15 |
| 6.4 | 1,30 | 1,37 |
| 6.9 | 0,65 | 0,80 |
| 7.0 | 0,57 | 0,75 |

Table 14   (continued)

| | $r_1$ (s$^{-1}$ mM$^{-1}$) | |
|---|---|---|
| pH | 20 minutes incubation | 2.0 hours incubation |
| 7.5 | 0,55 | 0,72 |
| 7.9 | 0,56 | 0,87 |
| 8.2 | 0,60 | 1,00 |

## Example 16

**pH - $r_1$ dependence of DPPE/DPSG (7:3 mole ratio) liposomal GdDTPA-BMA preincubated in human whole blood.**

DPPE (dipalmitoyl-glycero-3-phosphatidylethanolamine) / DPSG (dipalmitoylsuccinylglycerol) liposomes (7: 3 mole ratio) containing GdDTPA-BMA were prepared according to the method described in Example 1. The mean hydrodynamic diameter of the liposomes was measured to 110 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 15 and Fig. 15 show the pH-$r_1$ dependence of DPPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 15

| | $r_1$ (s$^{-1}$ mM$^{-1}$) | |
|---|---|---|
| pH | 20 minutes incubation | 60 minutes incubation |
| 5.7 | 2,00 | 2,16 |
| 6.0 | 1,45 | 1,59 |
| 6.4 | 0,92 | 0,99 |
| 6.9 | 0,67 | 0,74 |
| 7.4 glucose* | 0,59 | 0,62 |

*Dilution with glucose instead of buffer

## Example 17

**pH - $r_1$ dependence of DSPE/DPSG (9:1 mole ratio) liposomal GdDTPA-BMA in isosmotic buffer solutions**

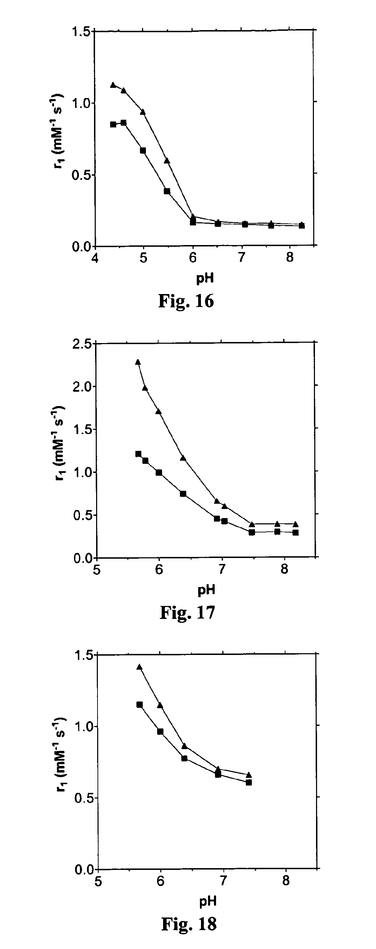

DSPE (distearoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 128 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(*N*-morpholino) ethanesulfonic acid (MES) buffers). The buffered liposome dispersions were incubated at 37 °C prior to the relaxation measurements. The investigated pH-range was 4.4 - 8.3. Table 16 and Fig. 16 show the pH-$r_1$ dependence of DSPE/DPSG liposomal Gd-DTPA-BMA at 37 °C in MES buffers after 20 minutes (■) and 2.0 (▲) hours incubation.

Table 16

| | $r_1$ (s$^{-1}$ mM$^{-1}$) | |
|---|---|---|
| pH | 20 minutes incubation | 2.0 hours incubation |
| 4.4 | 0,85 | 1,12 |

Table 16   (continued)

| pH | r$_1$ (s$^{-1}$ mM$^{-1}$) | |
| --- | --- | --- |
| | 20 minutes incubation | 2.0 hours incubation |
| 4.6 | 0,86 | 1,09 |
| 5.0 | 0,66 | 0,93 |
| 5.5 | 0,38 | 0,59 |
| 6.0 | 0,16 | 0,20 |
| 6.5 | 0,15 | 0,16 |
| 7.1 | 0,14 | 0,15 |
| 7.6 | 0,13 | 0,15 |
| 8.3 | 0,13 | 0,14 |

**Example 18**

**pH - r$_1$ dependence of DSPE/DPSG (9:1 mole ratio) liposomal GdDTPA-BMA in buffered human whole blood.**

DSPE (distearoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 128 nm as described in Example 1.The T$_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 17 and Fig. 17 show the pH-r$_1$ dependence of DSPE/DPSG liposomal Gd-DTPA-BMA at 37 °C in MES/HEPES buffered whole blood (50%) after 20 minutes (■) and 2.0 (▲) hours incubation. The r$_1$ values are corrected for the content of solid materials present in whole blood.

Table 17

| pH | r$_1$ (s$^{-1}$ mM$^{-1}$) | |
| --- | --- | --- |
| | 20 minutes incubation | 2.0 hours incubation |
| 5.7 | 1,21 | 2,28 |
| 5.8 | 1,13 | 1,98 |
| 6.0 | 0,99 | 1,71 |
| 6.4 | 0,74 | 1,16 |
| 6.9 | 0,45 | 0,65 |
| 7.0 | 0,42 | 0,60 |
| 7.5 | 0,29 | 0,38 |
| 7.9 | 0,29 | 0,38 |
| 8.2 | 0,28 | 0,38 |

**Example 19**

**pH - r$_1$ dependence of DSPE/DPSG (9:1 mole ratio) liposomal GdDTPA-BMA preincubated in human whole blood.**

DSPE (distearoyl-glycero-3-phosphatidylethanolamine) / DPSG (dipalmitoylsuccinylglycerol) liposomes (9:1 mole ratio) containing GdDTPA-BMA were prepared according to the method described in Example 1. The mean hydrodynamic diameter of the liposomes was measured to 128 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 18 and Fig. 18 show the pH-$r_1$ dependence of DSPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 18

| pH | $r_1$ (s$^{-1}$ mM$^{-1}$) | |
|---|---|---|
| | 20 minutes incubation | 60 minutes incubation |
| 5.7 | 1,15 | 1,41 |
| 6.0 | 0,96 | 1,14 |
| 6.4 | 0,77 | 0,86 |
| 6.9 | 0,65 | 0,69 |
| 7.4 glucose* | 0,60 | 0,65 |

*Dilution with glucose instead of buffer

## Example 20

### pH - $r_1$ dependence of DSPE/DPSG (7:3 mole ratio) liposomal GdDTPA-BMA in isosmotic buffer solutions

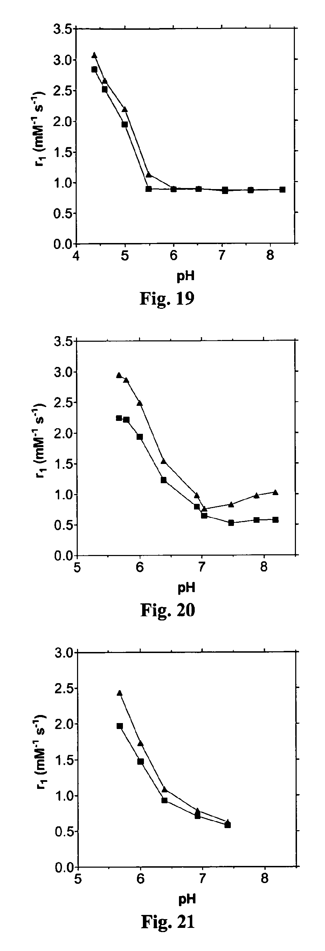

DSPE (distearoyl-glycero-3-phosphatidylethanolamine) / DPSG (dipalmitoylsuccinylglycerol) liposomes (7:3 mole ratio) containing GdDTPA-BMA were prepared according to the method described in Example 1. The mean hydrodynamic diameter of the liposomes was measured to 113 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(N-morpholino) ethanesulfonic acid (MES) buffers). The buffered liposome dispersions were incubated at 37 °C prior to the relaxation measurements. The investigated pH-range was 4.4 - 8.3. Table 19 and Fig. 19 show the pH-$r_1$ dependence of DSPE/DPSG liposomal Gd-DTPA-BMA at 37 °C in MES buffers after 20 minutes (■) and 2.0 (▲) hours incubation.

Table 19

| pH | $r_1$ (s$^{-1}$ mM$^{-1}$) | |
|---|---|---|
| | 20 minutes incubation | 2.0 hours incubation |
| 4.4 | 2,84 | 3,08 |
| 4.6 | 2,52 | 2,66 |
| 5.0 | 1,94 | 2,19 |
| 5.5 | 0,89 | 1,13 |
| 6.0 | 0,88 | 0,90 |
| 6.5 | 0,88 | 0,89 |
| 7.1 | 0,87 | 0,85 |
| 7.6 | 0,86 | 0,87 |
| 8.3 | 0,87 | 0,87 |

## Example 21

### pH - $r_1$ dependence of DSPE/DPSG (7:3 mole ratio) liposomal GdDTPA-BMA in buffered human whole blood.

DSPE (distearoyl-glycero-3-phosphatidylethanolamine) / DPSG (dipalmitoylsuccinylglycerol) liposomes (7:3 mole ratio) containing GdDTPA-BMA were prepared according to the method described in Example 1. The mean hydrodynamic diameter of the liposomes was measured to 113 nm as described in Example 1.The $T_1$-relaxation times were measured at 37 °C and 0.47 Tesla (Minispec PC-120b, Bruker GmbH, Rheinstetten, Germany) in different buffer solutions (0.25 M MES and 0.25 M N-2-hydroxyethylpiperazine-N'-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 20 and Fig. 20 show the pH-$r_1$ dependence of DSPE/DPSG liposomal Gd-DTPA-BMA at 37 °C in MES/HEPES buffered whole blood (50%) after 20 minutes (■) and 2.0 (▲) hours incubation. The $r_1$ values are corrected for the content of solid materials present in whole blood.

Table 20

| pH | $r_1$ (s$^{-1}$ mM$^{-1}$) | |
|---|---|---|
| | 20 minutes incubation | 2.0 hours incubation |
| 5.7 | 2,24 | 2,94 |
| 5.8 | 2,21 | 2,86 |
| 6.0 | 1,93 | 2,49 |
| 6.4 | 1,22 | 1,54 |
| 6.9 | 0,79 | 0,98 |
| 7.0 | 0,64 | 0,76 |
| 7.5 | 0,52 | 0,83 |
| 7.9 | 0,57 | 0,97 |
| 8.2 | 0,57 | 1,02 |

**Example 22**

**pH - $r_1$ dependence of DSPE/DPSG (7:3 mole ratio) liposomal GdDTPA-BMA preincubated in human whole blood.**

DSPE (distearoyl-glycero-3-phosphatidylethanolamine) / DPSG (dipalmitoylsuccinylglycerol) liposomes (7:3 mole ratio) containing GdDTPA-BMA were prepared according to the method described in Example 1. The mean hydrodynamic diameter of the liposomes was measured to 113 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 21 and Fig. 21 show the pH-$r_1$ dependence of DSPE/DPSG liposomal Gd-DTPA-BMA at 37 °C after preincubation in human whole blood and subsequently further incubation with MES/HEPES buffers (and one vial with glucose) for 20 minutes (■) and 60 (▲) minutes. The $r_1$ values are corrected for the content of solid materials present in whole blood.

Table 21

| pH | $r_1$ (s$^{-1}$ mM$^{-1}$) | |
|---|---|---|
| | 20 minutes incubation | 60 minutes incubation |
| 5.7 | 1,97 | 2,43 |
| 6.0 | 1,47 | 1,73 |
| 6.4 | 0,92 | 1,08 |
| 6.9 | 0,70 | 0,78 |
| 7.4 glucose* | 0,58 | 0,62 |

*Dilution with glucose instead of buffer

**Example 23**

***In vitro* MR imaging studies with DPPE/DPSG liposomal GdDTPA-BMA.**

DPPE/DPSG liposomes (4:1 mole ratio) containing GdDTPA-BMA were prepared according to the method described in Example 1. The mean hydrodynamic diameter of the liposomes was measured to 115 nm as described in Example 1. The MR contrast efficacy of the liposomal system was investigated in human whole blood in two gel phantoms at 1.0 T on a Siemens Magnetom Harmony system (Siemens AG, Erlangen, Germany). Each phantom was composed of eight glass vials placed equidistantly from each other and from the centre of a circular glass container. The latter was filled with an agar gel (2 % w/w) doped with GdDTPA-BMA to give a $T_1$ of about 500 ms at 1.0 T. Imaging was performed in a quadrature head coil. Quantitative $T_1$ maps were obtained from spoiled 2D gradient echo images (TR/TE/flip = 2.1 ms/ 1.7 ms/ 8°) prepared with a nonselective 180° inversion pulse. The time between the inversion pulse and the acquisition (TI) was varied between 220 ms and 5788 ms, with a total of 11 TI values used. The system gain was kept constant between consecutive scans so that the MR signal was directly comparable between scans. Other scan parameters were: voxel size: 1.17 x 1.17 x 9.0 mm$^3$, number of averages: 20. The inter-scan interval was 6 s to allow for almost complete recovery of the longitudinal magnetisation between scans. The resulting magnetisation recovery curve obtained from the 11 magnetisation prepared scans were fit to the equation:

$$SI(TI) = A \cdot \left|1\text{-}2\exp(\text{-}TI \cdot R_1)\right| + B \qquad [2]$$

where ‖ denotes the absolute value, A and B are scaling constants, TI is the inversion time and $R_1$ is the $T_1$ relaxation rate (s$^{-1}$). A Lovenberg-Marquard nonlinear least squares fit routine was used and parametric $R_1$ maps were generated on a pixel-by-pixel basis from the raw inversion recovery datasets. The resulting $R_1$ value of each liposome containing glass vial was measured from a constant diameter region of interest (ROI) placed in each vial in the image. The $C_{eff}$ in the samples was approximately 1 mM Gd after dilution (see below for details).

*Effect of Incubation in Buffered Human Whole Blood*

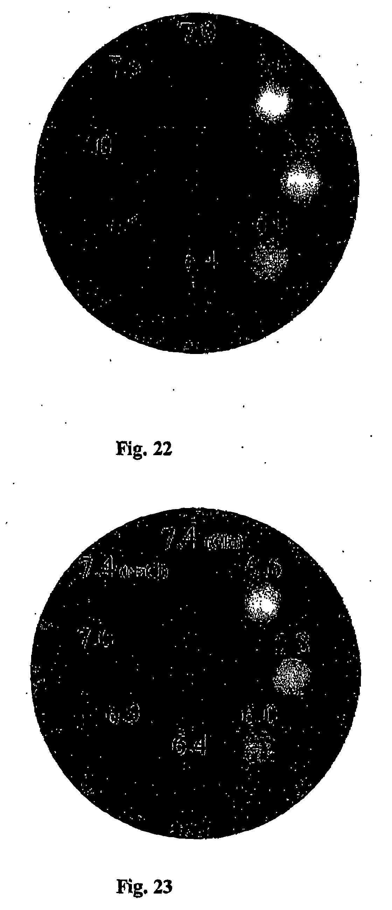

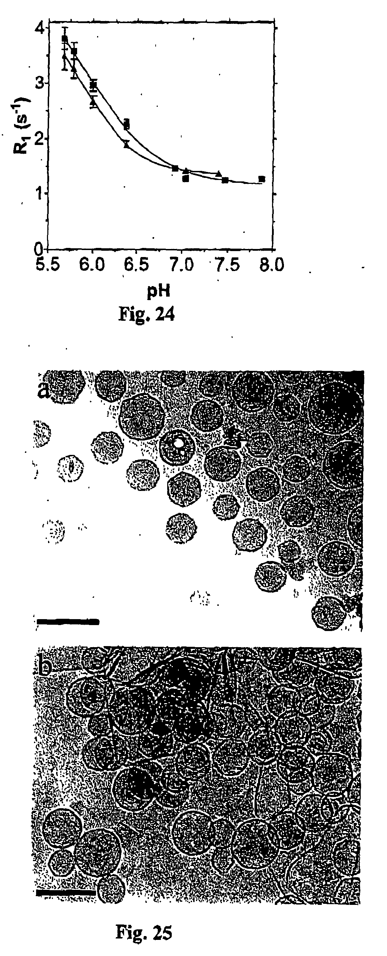

The liposomes were diluted with MES/HEPES (250 mM) buffered whole blood in the pH range 5.6-7.9. The volume ratios between liposome dispersion, buffer and human whole blood were 1/5/5, respectively. The samples were incubated at 37 °C for 20 minutes. The liposome dispersions were then transferred to the respective vials in the first phantom and equilibrated to room temperature prior to the MR imaging. Fig. 22 shows a quantitative $R_1$ map of the phantom. Fig. 24 shows the change in $R_1$ as a function of pH as measured from the ROI's placed in the parametric $R_1$ map (Data points: ■).

*Effect of Pre-incubation in Human Whole Blood*

The liposomes were diluted with whole blood and incubated at 37 °C for 20 minutes, followed by pH adjustment with 250 mM MES/HEPES buffers and further incubation for 20 minutes. Isotonic NaCl- and glucose-solution was added to two of the vials, respectively, instead of buffer, as controls at pH 7.4 (unaltered pH). The investigated pH range was 5.6 - 7.4. The volume ratios between liposome dispersion, human whole blood and buffer were 1/5/5, respectively. The dispersions were transferred to the respective vials in the second phantom and equilibrated to room temperature prior to the MR imaging. Fig. 23 shows a quantitative $R_1$ map of the phantom. Fig. 24 shows the change in $R_1$ as a function of pH as measured from the ROI's placed in the parametric $R_1$ map (Data points: ▲).

**Example 24**

**Cryo-transmission electron microscopy (Cryo-TEM) of DPPE/DPSG liposomal GdDTPA-BMA.**

DPPE/DPSG liposomes (4:1 mole ratio) containing GdDTPA-BMA were prepared according to the method described in Example 1. The mean hydrodynamic diameter of the liposomes was measured to 111 nm as described in Example 1. The DPPE/DPSG liposomal dispersion was diluted 3/1 and 1/1 (v/v) with isoosmotic buffer solutions (50 mM MES buffers) at pH 4.4 and 7.4, respectively. Different dilution factors were used at the two pH values in order to obtain micrographs with similar material content. The buffered liposomal dispersion at pH 4.4 was incubated at 37 °C for 30 minutes prior to the Cryo-TEM procedure, while the corresponding sample at pH 7.4 was assessed directly after dilution.

The Cryo-TEM preparation procedure consisted, in short, of the following: The dispersions described above were equilibrated at 25 °C and 98-99 % relative humidity within a climate chamber. A droplet ($\sim$2 $\mu$l) of sample dispersion was deposited on glow discharge treated 300 mesh copper grids (Agar Scientific Ltd, Stansted Essex, England) covered with a perforated polymer film coated with evaporated carbon on both sides. Excess liquid was removed with filter paper, followed by immediate plunging of the grid into liquid ethane held just above its freezing point. The vitrified sample was then transferred under protection against atmospheric conditions to a Zeiss EM 902A electron microscope (LEO Electron Microscopy, Oberkochen, Germany). The temperature of the specimen was kept below -165 °C during the whole process and all observations were made in zero loss brightfield mode and at an accelerating voltage of 80 kV. A minimum/micro-dose focussing system (MDF) was used to minimise radiation damage. The images were acquired by employing a cooled slow-scan CCD camera (Proscan GmbH, Scheuring, Germany), and analySIS software (Soft Imaging System GmbH, Münster, Germany). Figure 25 shows cryo-TEM micrographs of the DPPE/DPSG system at pH 7.4 (Fig. 25a) and after 30 minutes incubation at pH 4.4 and 37 °C (Fig. 25b). Well-separated unilamellar liposomes were mainly observed at pH 7.4, but small bilayer fragments were also present (Fig. 25a). Aggregated structures were present at pH 4.4, along with large bilayers (Fig. 25b). The white spots in the micrographs are irradiation-damaged areas.

### Example 25

**pH - $r_1$ dependence of DPPE/DPSG/DPPE-PEG2000 (4:1:0.025 mole ratio) liposomal GdDTPA-BMA in isosmotic buffer solutions**

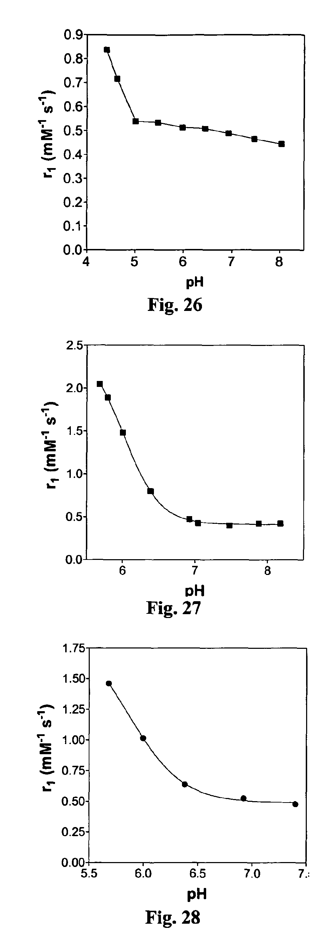

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.025 mole ratio) containing GdDTPA-BMA were prepared according to the method described in Example 1. The mean hydrodynamic diameter of the liposomes was measured to 111 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-($N$-morpholino) ethanesulfonic acid (MES) buffers). The buffered liposome dispersions were incubated at 37 °C prior to the relaxation measurements. The investigated pH-range was 4.4 - 8.3. Table 22 and Fig. 26 show the pH-$r_1$ dependence of DPPE/DPSG/DPPE-PEG2000 liposomal Gd-DTPA-BMA at 37 °C in MES buffers after 20 minutes (■) incubation.

Table 22

| pH | $r_1$ (s$^{-1}$ mM$^{-1}$) 20 minutes incubation |
|---|---|
| 4.4 | 0,83 |
| 4.6 | 0,71 |
| 5.0 | 0,53 |
| 5.5 | 0,53 |
| 6.0 | 0,51 |
| 6.5 | 0,50 |
| 7.1 | 0,48 |
| 7.6 | 0,46 |
| 8.3 | 0,44 |

### Example 26

**pH - $r_1$ dependence of DPPE/DPSG/DPPE-PEG2000 (4:1:0.025 mole ratio) in buffered human whole blood.**

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.025 mole ratio) containing GdDTPA-BMA were prepared according to the method described in Example 1. The mean hydrodynamic diameter of the liposomes was measured to 111 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 23 and Fig. 27 show the pH-$r_1$ dependence of DPPE/DPSG/DPPE-PEG2000 liposomal Gd-DTPA-BMA at 37 °C in MES/HEPES buffered whole blood (50%) after 20 minutes (■) incubation. The $r_1$ values are corrected for the content of solid materials present in whole blood.

Table 23

|  | $r_1$ (s$^{-1}$ mM$^{-1}$) |
|---|---|
| pH | 20 minutes incubation |
| 5.7 | 2,04 |
| 5.8 | 1,89 |
| 6.0 | 1,48 |
| 6.4 | 0,79 |
| 6.9 | 0,47 |
| 7.0 | 0,42 |
| 7.5 | 0,40 |
| 7.9 | 0,42 |
| 8.2 | 0,42 |

**Example 27**

**pH - $r_1$ dependence of DPPE/DPSG/DPPE-PEG2000 (4:1:0.025 mole ratio) preincubated in human whole blood.**

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.025 mole ratio) containing GdDTPA-BMA were prepared according to the method described in Example 1. The mean hydrodynamic diameter of the liposomes was measured to 111 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 24 and Fig. 28 show the pH-$r_1$ dependence of DPPE/DPSG/DPPE-PEG2000 liposomal Gd-DTPA-BMA at 37 °C after preincubation in human whole blood and subsequently further incubation with MES/HEPES buffers (and one vial with glucose) for 20 minutes (■). The $r_1$ values are corrected for the content of solid materials present in whole blood.

Table 24

|  | $r_1$ (s$^{-1}$ mM$^{-1}$) |
|---|---|
| pH | 20 minutes incubation |
| 5.7 | 1,46 |
| 6.0 | 1,01 |
| 6.4 | 0,63 |
| 6.9 | 0,52 |
| glucose* | 0,47 |

*Dilution with glucose instead of buffer

**Example 28**

**pH - $r_1$ dependence of DPPE/DPSG/DPPE-PEG2000 (4:1:0.076 mole ratio) in buffered human whole blood.**

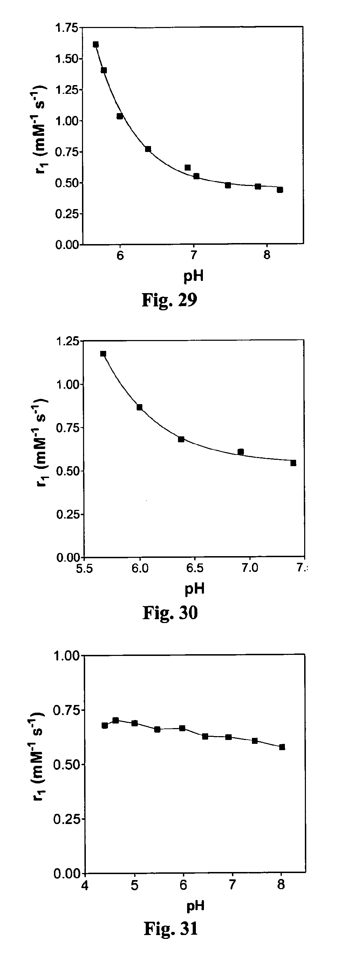

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.076 mole ratio) containing GdDTPA-BMA were prepared according to the method described in Example 1. The mean hydrodynamic diameter of the liposomes was measured to 106 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M $N$-2-hydroxyethylpiperazine-$N'$-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 25 and Fig. 29 show the pH-$r_1$ dependence of DPPE/DPSG/DPPE-PEG2000 liposomal Gd-DTPA-BMA at 37 °C in MES/HEPES buffered whole blood (50%) after 20 minutes (■) incubation. The $r_1$ values are corrected for the content of solid materials present in whole blood.

Table 25

| pH | $r_1$ (s$^{-1}$ mM$^{-1}$) |
|-----|-----|
| | 20 minutes incubation |
| 5.7 | 1,61 |
| 5.8 | 1,40 |
| 6.0 | 1,03 |
| 6.4 | 0,77 |
| 6.9 | 0,62 |
| 7.0 | 0,55 |
| 7.5 | 0,47 |
| 7.9 | 0,46 |
| 8.2 | 0,43 |

**Example 29**

**pH - $r_1$ dependence of DPPE/DPSG/DPPE-PEG2000 (4:1:0.076 mole ratio) preincubated in human whole blood.**

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.076 mole ratio) containing GdDTPA-BMA were prepared according to the method described in Example 1. The mean hydrodynamic diameter of the liposomes was measured to 106 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 26 and Fig. 30 show the pH-$r_1$ dependence of DPPE/DPSG/DPPE-PEG2000 liposomal Gd-DTPA-BMA at 37 °C after preincubation in human whole blood and subsequently further incubation with MES/HEPES buffers (and one vial with glucose) for 20 minutes (■). The $r_1$ values are corrected for the content of solid materials present in whole blood.

Table 26

| pH | $r_1$ (s$^{-1}$ mM$^{-1}$) |
|-----|-----|
| | 20 minutes incubation |
| 5.7 | 1,17 |
| 6.0 | 0,86 |
| 6.4 | 0,68 |

Table 26   (continued)

|  | $r_1$ (s$^{-1}$ mM$^{-1}$) |
|---|---|
| pH | 20 minutes incubation |
| 6.9 | 0,60 |
| glucose* | 0,54 |

*Dilution with glucose instead of buffer

## Example 30

### pH - $r_1$ dependence of DPPE/DPSG/DPPE-PEG2000 (4:1:0.13 mole ratio) liposomal GdDTPA-BMA in isosmotic buffer solutions

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.13 mole ratio) containing GdDTPA-BMA were prepared according to the method described in Example 1. The mean hydrodynamic diameter of the liposomes was measured to 112 nm as described in Example 1. The $T_1$-relaxation times were measured at 37 °C and 0.47 Tesla (Minispec PC-120b, Bruker GmbH, Rheinstetten, Germany) in different isosmotic buffer solutions (0.05 M 2-(*N*-morpholino) ethanesulfonic acid (MES) buffers). The buffered liposome dispersions were incubated at 37 °C prior to the relaxation measurements. The investigated pH-range was 4.4 - 8.3. Table 27 and Fig. 31 show the pH-$r_1$ dependence of DPPE/DPSG/DPPE-PEG2000 liposomal Gd-DTPA-BMA at 37 °C in MES buffers after 20 minutes (■) incubation.

Table 27

|  | $r_1$ (s$^{-1}$ mM$^{-1}$) |
|---|---|
| pH | 20 minutes incubation |
| 4.4 | 0,67 |
| 4.6 | 0,70 |
| 5.0 | 0,68 |
| 5.5 | 0,66 |
| 6.0 | 0,66 |
| 6.5 | 0,62 |
| 7.1 | 0,62 |
| 7.6 | 0,60 |
| 8.3 | 0,57 |

## Example 31

### pH - $r_1$ dependence of DPPE/DPSG/DPPE-PEG2000 (4:1:0.13 mole ratio) in buffered human whole blood.

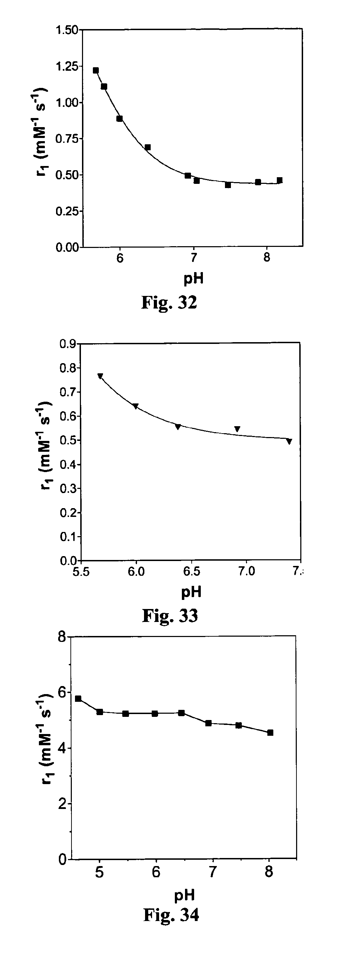

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.13 mole ratio) containing GdDTPA-BMA were prepared according to the method described in Example 1. The mean hydrodynamic diameter of the liposomes was measured to 112 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M *N*-2-hydroxyethylpiperazine-*N'*-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 28 and Fig. 32 show the pH-$r_1$ dependence of DPPE/DPSG/DPPE-PEG2000 liposomal Gd-DTPA-BMA at 37 °C in MES/HEPES buffered whole blood (50%) after 20 minutes (■) incubation. The $r_1$ values are corrected for the content of solid materials present in whole blood.

Table 28

| | $r_1$ (s$^{-1}$ mM$^{-1}$) |
|---|---|
| pH | 20 minutes incubation |
| 5.7 | 1,22 |
| 5.8 | 1,10 |
| 6.0 | 0,88 |
| 6.4 | 0,68 |
| 6.9 | 0,49 |
| 7.0 | 0,45 |
| 7.5 | 0,42 |
| 7.9 | 0,44 |
| 8.2 | 0,45 |

**Example 32**

**pH - $r_1$ dependence of DPPE/DPSG/DPPE-PEG2000 (4:1:0.13 mole ratio) preincubated in human whole blood.**

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.13 mole ratio) containing GdDTPA-BMA were prepared according to the method described in Example 1. The mean hydrodynamic diameter of the liposomes was measured to 112 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 29 and Fig. 33 show the pH-$r_1$ dependence of DPPE/DPSG/DPPE-PEG2000 liposomal Gd-DTPA-BMA at 37 °C after preincubation in human whole blood and subsequently further incubation with MES/HEPES buffers (and one vial with glucose) for 20 minutes (■). The $r_1$ values are corrected for the content of solid materials present in whole blood.

Table 29

| | $r_1$ (s$^{-1}$ mM$^{-1}$) |
|---|---|
| pH | 20 minutes incubation |
| 5.7 | 0,76 |
| 6.0 | 0,63 |
| 6.4 | 0,55 |
| 6.9 | 0,54 |
| glucose* | 0,48 |

*Dilution with glucose instead of buffer

**Example 33**

**pH - $r_1$ dependence of DPPE/DPSG (4:1 mole ratio) liposomal Gadofosveset Trisodium in isosmotic buffer solutions**

DPPE (dipalmitoyl-glycero-3-phosphatidylethanolamine) / DPSG (dipalmitoylsuccinylglycerol) liposomes (4:1 mole ratio) containing Gadofosveset Trisodium (synthesised as described in WO-A-96/23526) were prepared according to the method described in Example 1. The hydration was performed with an aqueous solution containing 20 mM Gadofosveset Trisodium and 50 mM TRIS/HCl buffer (pH 8.4). The mean hydrodynamic diameter of the liposomes was measured to 117 nm as described in Example 1. The $T_1$-relaxation times were measured at 37 °C and 0.47 Tesla (Minispec PC-120b, Broker GmbH, Rheinstetten, Germany) in different isosmotic buffer solutions (0.05 M 2-($N$-morpholino) ethanesulfonic acid (MES) buffers). The buffered liposome dispersions were incubated at 37 °C prior to the relaxation measurements. The investigated pH-range was 4.4 - 8.3. Table 30 and Fig. 34 show the pH-$r_1$ dependence of DPPE/DPSG liposomal Gadofosveset Trisodium at 37 °C in MES buffers after 20 minutes (■) incubation.

Table 30

| pH | $r_1$ (s$^{-1}$ mM$^{-1}$) |
|---|---|
| | 20 minutes incubation |
| 4.4 | 5.76 |
| 4.6 | 5.78 |
| 5.0 | 5.29 |
| 5.5 | 5.23 |
| 6.0 | 5.23 |
| 6.5 | 5.25 |
| 7.1 | 4.87 |
| 7.6 | 4.79 |
| 8.3 | 4.51 |

**Example 34**

**pH - $r_1$ dependence of DPPE/DPSG (4:1 mole ratio) liposomal Gadofosveset Trisodium in buffered human whole blood.**

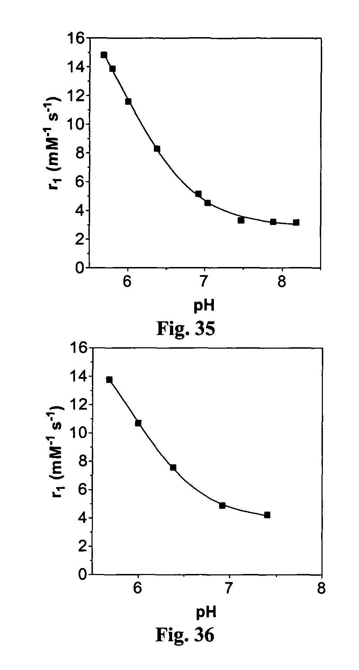

DPPE (dipalmitoyl-glycero-3-phosphatidylethanolamine) / DPSG (dipalmitoylsuccinylglycerol) liposomes (4:1 mole ratio) containing Gadofosveset Trisodium were prepared according to the method described in Example 1. The hydration was performed with an aqueous solution containing 20 mM Gadofosveset Trisodium and 50 mM TRIS/HCl buffer (pH 8.4). The mean hydrodynamic diameter of the liposomes was measured to 117 nm as described in Example 1. The $T_1$-relaxation times were measured at 37 °C and 0.47 Tesla (Minispec PC-120b, Bruker GmbH, Rheinstetten, Germany) in different buffer solutions (0.25 M MES and 0.25 M $N$-2-hydroxyethylpiperazine-$N'$-2-ethanesulfonic acid (HEPES) buffers) containing 50 volume % human whole blood. The buffered liposome dispersions were incubated at 37 °C prior to the relaxation measurements. The investigated pH-range was 5.7 - 8.2. Table 31 and Fig. 35 show the pH-$r_1$ dependence of DPPE/DPSG liposomal Gadofosveset Trisodium at 37 °C in MES/HEPES buffered whole blood (50%) after 20 minutes (■) incubation. The $r_1$ values are corrected for the content of solid materials present in whole blood.

Table 31

| pH | $r_1$ (s$^{-1}$ mM$^{-1}$) |
|---|---|
| | 20 minutes incubation |
| 5.7 | 14,82 |
| 5.8 | 13,87 |
| 6.0 | 11,58 |
| 6.4 | 8,29 |
| 6.9 | 5,155 |
| 7.0 | 4,53 |
| 7.5 | 3,32 |
| 7.9 | 3,23 |
| 8.2 | 3,17 |

**Example 35**

**pH - $r_1$ dependence of DPPE/DPSG (4:1 mole ratio) liposomal Gadofosveset Trisodium preincubated in human whole blood.**

DPPE (dipalmitoyl-glycero-3-phosphatidylethanolamine) / DPSG (dipalmitoylsuccinylglycerol) liposomes (4: 1 mole ratio) containing Gadofosveset Trisodium were prepared according to the method described in Example 1. The hydration was performed with an aqueous solution containing 20 mM Gadofosveset Trisodium and 50 mM TRIS/HCl buffer (pH 8.4). The mean hydrodynamic diameter of the liposomes was measured to 117 nm as described in Example 1. The liposomes were incubated for 20 minutes at 37 °C in human whole blood without pH adjustment. The pH in the vials were then adjusted by adding 0.25 M MES and 0.25 M HEPES buffers and one vial with glucose, and subsequently incubated further at 37 °C. The vials contained 50 volume % whole blood and the investigated pH-range was 5.7 - 7.5. $T_1$-relaxation times were measured at 37 °C and 0.47 Tesla (Minispec PC-120b, Bruker GmbH, Rheinstetten, Germany). Table 32 and Fig. 36 show the pH-$r_1$ dependence of DPPE/DPSG liposomal Gadofosveset Trisodium at 37 °C after preincubation in human whole blood and subsequently further incubation with MES/HEPES buffers (and one vial with glucose) for 20 minutes (■). The $r_1$ values are corrected for the content of solid materials present in whole blood.

Table 32

|  | $r_1$ (s$^{-1}$ mM$^{-1}$) |
| --- | --- |
| pH | 20 minutes incubation |
| 5.7 | 13,77 |
| 6.0 | 10,69 |
| 6.4 | 7,58 |
| 6.9 | 4,89 |
| glucose* | 4,23 |

*Dilution with glucose instead of buffer

**Example 36**

**Biodistribution of DPPE/DPSG (4:1 mole ratio) liposomal GdDTPA-BMA in rats**

DPPE (dipalmitoyl-glycero-3-phosphatidylethanolamine) / DPSG (dipalmitoylsuccinylglycerol) liposomes (4: 1 mole ratio) containing GdDTPA-BMA were prepared according to the method described in Example 1. The mean hydrodynamic diameter of the liposomes was measured to 113 nm as described in Example 1. The liposome formulation was intravenous injected to male BkI:SD rats (B&K Universal, Sweden) at a single dosage of 30 μmol Gd(III)/kg body weight. The injection rate was 1.2 ml/minute. The rats were anaesthetised to surgical level by subcutaneous administration of a 1:1 mixture of fentanyl/fluanisone (Hypnorm®, Janssen Pharmaceutica BV, Beerse, Belgium) and midazolam (Dormicum®, F. Hoffinann-La Roche AG, Basel, Switzerland) at time points varying from five minutes to four hours after injection (n=3). The rats were killed by section of the vena cava and blood was withdrawn into heparin-coated tubes. Liver, spleen and lungs were excised and freeze-stored for later ICP-AES analysis. Table 33 shows the tissue uptake in percent of administered dose as a function of time after administration of DPPE/DPSG liposomal GdDTPA-BMA. Data are given as the arithmetic mean ± SEM (n=3).

Table 33

| Time post Injection (min) | Tissue uptake (% of administered dose) | | | |
| --- | --- | --- | --- | --- |
| | Blood | Liver | Lungs | Spleen |
| 5 | 26.6±3.7 | 39.5±10.1 | 2.7±0.1 | 5.6±2.7 |
| 15 | 16.1±2.0 | 53.8±5.8 | 2.9±0.3 | 16.4±2.1 |
| 30 | 3.5±0.6 | 73.4±2.6 | 1.1±0.2 | 14.7±2.5 |
| 60 | 0.8±0.3 | 57.6±3.1 | 0.5±0.2 | 9.7±3.3 |

Table 33 (continued)

| Time post Injection (min) | Tissue uptake (% of administered dose) | | | |
|---|---|---|---|---|
| | **Blood** | **Liver** | **Lungs** | **Spleen** |
| 120 | 0.4±0.1 | 54.8±8.3 | 0.5±0.2 | 11.0±0.5 |

**Example 37**

**Biodistribution of DPPE/DPSG/DPPE-PEG2000 (4:1:0.076 mole ratio) liposomal GdDTPA-BMA in rats**

DPPE (dipalmitoyl-glycero-3-phosphatidylethanolamine) / DPSG (dipalmitoylsuccinylglycerol) / DPPE-PEG2000 (dipalmitoyl-glycero-3-phosphatidylethanolamine-polyethyleneglycol 2000) liposomes (4:1:0.076 mole ratio) containing GdDTPA-BMA were prepared according to the method described in Example 1. The mean hydrodynamic diameter of the liposomes was measured to 111 nm as described in Example 1. The liposome formulation was intravenous injected to male Bkl:SD rats (B&K Universal, Sweden) at a single dosage of 30 µmol Gd(III)/kg body weight. The injection rate was 1.2 ml/minute. The rats were anaesthetised to surgical level by subcutaneous administration of a 1:1 mixture of fentanyl/fluanisone (Hypnorm®, Janssen Pharmaceutica BV, Beerse, Belgium) and midazolam (Dormicum®, F. Hoffmann-La Roche AG, Basel, Switzerland) at time points varying from five minutes to four hours after injection (n=3). The rats were killed by section of the vena cava and blood was withdrawn into heparin-coated tubes. Liver, spleen and lungs were excised and freeze-stored for later ICP-AES analysis. Table 34 shows the tissue uptake in percent of administered dose as a function of time after administration of DPPE/DPSG/DPPE-PEG2000 liposomal GdDTPA-BMA. Data are given as the arithmetic mean ± SEM (n=3).

Table 34

| Time post Injection (min) | Tissue uptake (% of administered dose) | | | |
|---|---|---|---|---|
| | **Blood** | **Liver** | **Lungs** | **Spleen** |
| 5 | 67.1±7.0 | 19.8±10.3 | 0.8±0.3 | 0.6±0.3 |
| 30 | 42.7±2.1 | 23.7±1.0 | 0.5±0.1 | 0.6±0.0 |
| 60 | 27.1±2.4 | 39.2±2.1 | 0.4±0.1 | 2.1±0.1 |
| 120 | 4.2±0.4 | 49.5±2.7 | 0.1±0.0 | 2.4±0.1 |
| 240 | 0.6±0.1 | 49.1±2.8 | 0.1±0.1 | 2.4±0.1 |

**Claims**

1. A pH-sensitive liposome system comprising

   (a) 1-acyl-2-acyl-glycero-3-phosphoethanolamine and
   (b) 1-acyl-2-acylsuccinylglycerol or 1-acyl-3-acylsuccinylglycerol

   wherein in (a), both acyl-residues are the same or different and are saturated acyl residues that contain at least 14 C atoms or,
   one acyl residue is a saturated acyl residue that contains at least 14 C atoms and the other acyl residue is a monounsaturated acyl residue that contains at least 14 C atoms and,
   wherein in (b), both acyl residues are the same or different and are saturated acyl residues that contain at least 14 C atoms or,
   one acyl residue is a saturated acyl residue that contains at least 14 C atoms and the other acyl residue is a monounsaturated acyl residue that contains at least 14 C atoms.

2. The pH-sensitive liposome system according to claim 1 wherein the acyl residues contain 14 - 22 C atoms.

3. The pH-sensitive liposome system according to claims 1 and 2 wherein in (a), both acyl residues are the same saturated acyl residues and in (b), both acyl residues are the same saturated acyl residues, the acyl residues in (a) and (b) being the same or different.

4. The pH-sensitive liposome system according to claims 1 to 3 wherein (a) is selected form the group consisting of dimyristoyl-glycero-3-phosphoethanolamine, dipentadecanoyl-glycero-3-phosphoethanolamine, dipalmitoyl-glycero-3-phosphoethanol-amine, diheptadecanoyl-glycero-3-phosphoethanolamine, distearoyl-glycero-3-phosphoethanolamine, dinonadecanoyl-glycero-3-phosphoethanolamine and diarachidoyl-glycero-3-phosphoethanolamine and (b) is selected from the group consisting of the 1,2- or 1,3-isomers of dimyristoylsuccinylglycerol, dipentadecanoylsuccinylglycerol, dipalmitoylsuccinylglycerol, diheptadecanoylsuccinylglycerol, distearylsuccinylglycerol, dinonadecanoylsuccinylglycerol and diarachidonylsuccinylglycerol.

5. The pH-sensitive liposome system according to any of the preceding claims 1 to 4, wherein 1-acyl-2-acyl-glycero-3-phosphoethanolamine (a) is partially substituted by the corresponding polyethylene glycol (PEG)-modified compound 1-acyl-2-acyl-glycero-3-phosphoethanolamine-PEG (a*).

6. The pH-sensitive liposome system according to claim 5 wherein (a*) is present in an amount of from 0.5 to 5 mol%.

7. A composition comprising the pH-sensitive liposome system according to claims 1 to 6 and at least one contrast generating species useful in MR imaging or spectroscopy or optical imaging.

8. A composition according to claim 7 wherein the contrast generating species is one that is useful in MR imaging or spectroscopy.

9. A composition according to claim 8 wherein the contrast generating species is selected from the group consisting of paramagnetic compounds, superparamagnetic compounds, ferrimagnetic compounds, ferromagnetic compound and compounds containing other non zero spin nuclei than hydrogen.

10. A composition according to claims 8 to 9 wherein the contrast generating species is a paramagnetic species selected from the group consisting of free radicals, compounds comprising transition metals and compounds comprising lanthanide metals, preferably transition metal chelates, transition metal salts and lanthanide metal chelates, more preferably Gd-chelates, Dy-chelates, Eu-chelates Tm-chelates, Mn-chelates and manganese salts.

11. A composition according to claim 7 wherein the contrast generating species is a dye useful in optical imaging.

12. Composition according to claims 7 to 11 for use as a contrast agent.

13. A method for the identification of a tumour, inflammation or cardiac infarct tissue in a human or non-human animal subject comprising

    a) administering to said human or non-human animal subject a contrast agent comprising the composition according to claims 7 to 11, and
    b) generating at least one MR image, optical image or MR spectrum of said subject.

14. Use of the composition according to claims 7 to 11 for the manufacture of a contrast agent for the identification of a tumour, inflammation or cardiac infarct tissue in a human or non-human animal subject by MR imaging, optical imaging or MR spectroscopy.

15. A composition comprising the pH-sensitive liposome system according to claims 1 to 6 and at least one therapeutic agent.

16. A composition according to claim 15 wherein the therapeutic agent is an agent useful in tumour therapy or useful in the treatment of inflammation.

17. Compositions according to claims 15 to 16 for use as a medicament.

18. Use of the composition according to claims 15 to 16 for the manufacture of a medicament for the treatment of tumours or inflammation.

19. A composition according to claims 15 to 16 further comprising at least one contrast generating species useful in MR imaging or spectroscopy, optical imaging, X-ray, PET or ultrasound imaging.

Fig. 1

Fig.2

Fig. 3

**Fig. 4**

**Fig. 5**

**Fig. 6**

Fig. 7

Fig. 8

Fig. 9

**Fig. 10**

**Fig. 11**

**Fig. 12**

**Fig. 13**

**Fig. 14**

**Fig. 15**

**Fig. 16**

**Fig. 17**

**Fig. 18**

**Fig. 19**

**Fig. 20**

**Fig. 21**

Fig. 22

Fig. 23

Fig. 24

Fig. 25

**Fig. 26**

**Fig. 27**

**Fig. 28**

Fig. 29

Fig. 30

Fig. 31

**Fig. 32**

**Fig. 33**

**Fig. 34**

Fig. 35

Fig. 36